# EUROPEAN PATENT SPECIFICATION

(11) **EP 0 402 644 B1**
(45) Date of publication and mention of the grant of the patent: **16.08.1995**
(21) Application number: 90109208.0
(22) Date of filing: 16.05.1990
(51) Int. Cl.: C07D 231/56, C07D 261/20, C07D 275/04, C07D 401/04, C07D 413/04, C07D 417/04, A61K 31/445, A61K 31/495

(54) **N-(aryloxyalkyl)heteroarylpiperidines and -heteroarylpiperazines,a process for their preparation and their use as medicaments**
N-(aryloxyalkyl)heteroarylpiperidine und -heteroarylpiperazine, Verfahren zu ihrer Herstellung und ihre Verwendung als Medikamente
N-(aryloxyalkyl)hétéroarylpipéridines et -hétéroarylpipérazines, leur procédé de préparation et leur application comme médicaments

(30) Priority: 19.05.1989 US 354411; 29.12.1989 US 456790
(43) Date of publication of application: 19.12.1990
(73) Proprietor: HOECHST-ROUSSEL PHARMACEUTICALS INCORPORATED, Somerville New Jersey 08876 (US)
(72) Inventor: Strupczewski, Joseph Thomas, Flemington, NJ 08822 (US); Helsley, Grover Cleveland, Pluckemin, NJ 07978 (US); Chiang, Yulin, Convent Station, NJ 07961 (US); Bordeau, Kenneth J., Upper Black Eddy, PA 18972 (US)
(74) Representative: Losert, Wolfgang Dr.

(56) References cited:
- EP-A- 0 135 781
- DE-A- 3 530 089
- US-A- 4 355 037
- US-A- 4 458 076

## Description

This invention relates to substituted N-(aryloxyalkyl)heteroarylpiperidines and heteroarylpiperazines having antipsychotic activity and to their use as antipsychotic drugs.

The therapeutic treatment of schizophrenic patients by administration of neuroleptic drugs, such is chlorpromazine, haloperidol, sulpiride, and chemically closely related compounds, is widespread. While control of schizophrenic symptoms has been successful, treatment with these drugs does not cure the psychotic patient, who will almost certainly relapse if medication is discontinued. There exists a continuing need in the art for antipsychotic drugs for the treatment of psychoses.

Moreover, some of the known neuroleptics produce unwanted side effects. For example, the side effects of many antipsychotic drugs include the so-called extrapyramidal symptoms, such as rigidity and tremor, continuous restless walking, facial grimacing, and involuntary movements of the face and extremities. Hypotension is also common. Thus, there also exists a need in the art for antipsychotic drugs that produce fewer or less severe manifestations of these common side effects.

Moreover, there has been a need for drugs that can produce other biological effects. For example, relief from pain has been an age-old aspiration which has led to the discovery of natural and synthetic analgetics. Nevertheless, the need for safe and effective analgetics has continued to the present day.

### SUMMARY OF THE INVENTION

This invention aids in fulfilling these needs in the art by providing a compound of the formula: 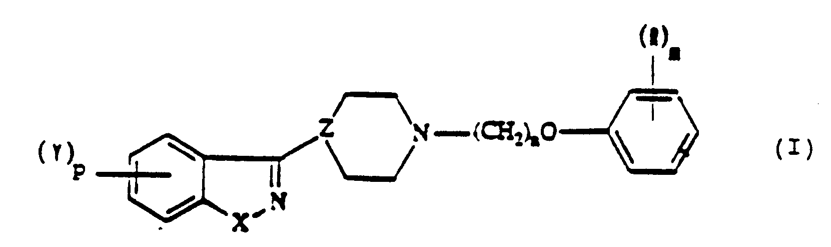
wherein
X is -O-, -S-, 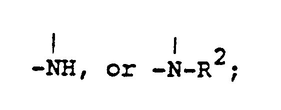
Y is hydrogen, C₁ ― C₆-alkyl, -OH, Cl, F, Br, I, C₁― C₆-alkoxy, -CF₃, -NO₂, or -NH₂, when p is 1;
Y is C₁ ― C₆-alkoxy when p is 2 and X is -O-;
R² is selected from the group consisting of lower alkyl, aryl lower alkyl, aryl, cycloalkyl, aroyl, alkanoyl, and phenyl sulfonyl groups;
Z is -CH- or 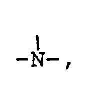
with the proviso that Z is not 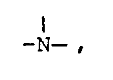
when
X is -S- and R is H;
n is 2, 3, 4, or 5;
R is hydrogen, alkyl, C₁ - C₆ alkoxy, hydroxyl, carboxyl, Cl, F, Br, I, amino, C₁ - C₆ mono or dialkyl amino, -NO₂, lower alkyl thio, -OCF₃, cyano, acylamino, -CF₃, trifluoroacetyl, aminocarbonyl, 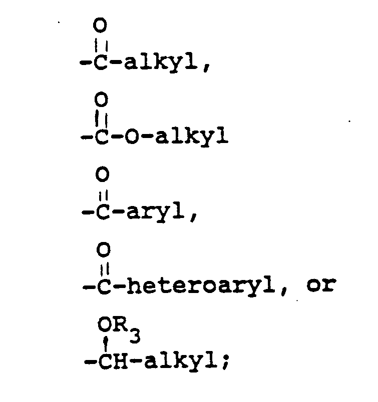
where alkyl is lower alkyl;
aryl is phenyl or 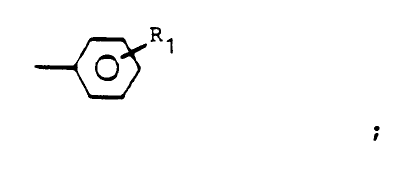
R₁ is hydrogen, lower alkyl, C₁ - C₆ alkoxy, hydroxy, Cl, F, Br, I, C₁ - C₆ alkyl amino, -NO₂, -CN, -CF₃, -OCF₃;
heteroaryl is 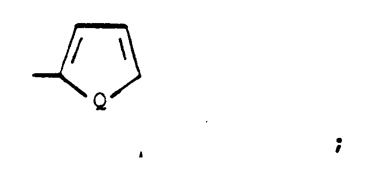
where
Q is -O-, -S-, 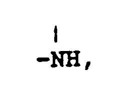
-CH=N;
m is 1, 2 or 3 with the proviso that when m is 2 or 3, R can be the same or different;
R₃ is hydrogen, lower alkyl, or acyl;
or a pharmaceutically acceptable acid addition salt thereof.

This invention also provides a pharmaceutical composition, which comprises a compound of the invention and a pharmaceutically acceptable carrier therefor. In one embodiment of the invention, the pharmaceutical composition is an antipsychotic composition comprising a compound of the invention in an amount sufficient to produce an antipsychotic effect.

### DETAILED DESCRIPTION OF THE PREFERRED EMBODIMENT

The compounds of this invention are useful as antipsychotic drugs and as analgesic agents. The compounds of the invention can contain a variety of different substituents and chemical groups. As used herein, when the term "lower" is mentioned in connection with the description of a particular group, the term means that the group it is describing contains from 1 to 6 carbon atoms.

The term "alkyl" as used herein refers to a straight or branched chain hydrocarbon group containing no unsaturation, for example, methyl, ethyl, isopropyl, 2-butyl, neopentyl, or n-hexyl.

The term "alkoxy" as used herein refers to a monovalent substituent comprising an alkyl group linked through an ether oxygen having its free valence bond from the ether oxygen, e.g. methoxy, ethoxy, propoxy, butoxy, or pentoxy.

The term "alkylene" as used herein refers to a bivalent radical of a lower branched or unbranched alkyl group having valence bonds on two terminal carbons thereof, for example, ethylene (-CH₂CH₂-), propylene (-CH₂CH₂CH₂-), or isopropylene

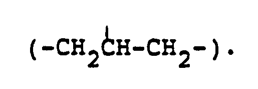

The term "cycloalkyl" refers to a saturated hydrocarbon group possessing at least one carbocyclic ring, the ring containing from 3 to 10 carbon atoms such as cyclopropyl, cyclobutyl, cyclopentyl, cyclohexyl, cycloheptyl, cyclodecyl and the like.

The term "alkanoyl" as used herein refers to an alkyl carbonyl moiety containing from 2 to 11 carbon atoms, e.g. 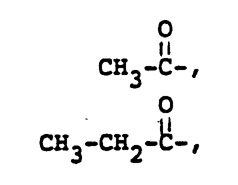
etc.

The term "alkanoic acid" refers to a compound formed by combination of a carboxyl group with a hydrogen atom or alkyl group. Examples of alkanoic acids are formic acid, acetic acid, propanoic acid, 2,2-dimethylacetic acid, hexanoic acid, octanoic acid, decanoic acid, and the like.

The term "alkanoyl" refers to the radical formed by removal of the hydroxyl function from an alkanoic acid. Examples of alkanoyl groups are formyl, acetyl, propionyl, 2,2-dimethylacetyl, hexanoyl, octanoyl, decanoyl and the like.

The term "aryl lower alkyl" refers to compounds wherein "aryl" and "loweralkyl" are as defined above.

The term "lower alkylthio" refers to a monovalent substituent having the formula lower alkyl-S-.

The term "phenylsulfonyl" refers to a monovalent substituent having the formula phenyl-SO₂-.

The term "acyl" refers to a substituent having the formula

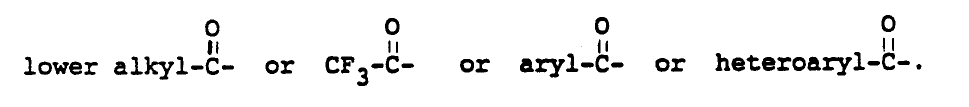

Unless otherwise indicated, the term "halogen" as used herein refers to a member of the halogen family selected from the group consisting of fluorine, chlorine, bromine, and iodine.

Throughout the specification and appended claims, a given chemical formula or name shall encompass all geometric and stereoisomers thereof where such isomers exist.

DE-A-3 530 089 discloses N-(phenyloxyalkyl)benzoisothiazolpiperazines and their pharmaceutical use as analgetics.

EP-A-135 781 discloses 3-(piperidinyl)- and 3-(pyrrolidinyl)-1H-indazoles and their pharmaceutical use as analgetics and antipsychotics.

### A. COMPOUNDS OF THE INVENTION

The compounds of this invention can be represented by the following formula: 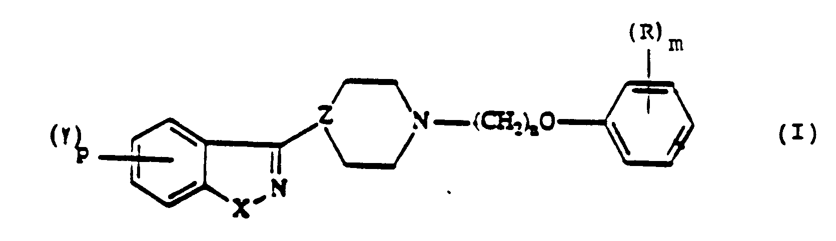
The substituent X in formula (I) is selected from the group consisting of -O-, -S-, -NH-, or 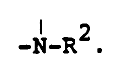
When the substituent X is -O-, the compounds of the invention contain a 1,2-benzisoxazole nucleus, and when X is -S-, the compounds of the invention contain a 1,2-benzisothiazole nucleus. When X is -NH- or 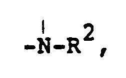
the compounds of the invention contain the indazole nucleus.

When p in formula (I) is 1, the substituent Y is selected from the group consisting of hydrogen, C₁-C₆-alkyl, hydroxyl, halogen, C₁-C₆-alkoxy, -CF₃, -NO₂, and -NH₂. The substituent Y is preferably in the 5- or 6-position of the ring. Moreover, in the preferred embodiments of the invention, the substituent Y is hydrogen, chlorine, or fluorine, and in the particularly preferred compounds of the invention, Y is fluorine, especially in the 6-position of the ring.

When p in formula (I) is 2 and X is -O-, each Y substituent can be independently selected from C₁-C₆-alkoxy groups, preferably methoxy groups.

The value of n in formula (I) can be 2, 3, 4, or 5, and preferably is 2, 3, or 4. In the particularly preferred compounds of the invention, n is 3.

When X in the compounds of the invention is 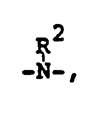
the substituent R² is selected from the group consisting of lower alkyl, aryl lower alkyl, aryl, cycloalkyl, aroyl, alkanoyl, and phenylsulfonyl groups.

The substituent Z in formula (I) can be 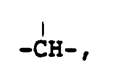
in which case the compounds of the invention are heteroarylpiperidine derivatives, or 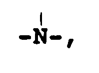
in which case the compounds are heteroarylpiperazine derivatives. The preferred compounds of the invention are the heteroarylpiperidines.

The compounds of the invention can contain one, two, or three R-substituents. The substituent R can be hydrogen, alkyl, C₁ - C₆ alkoxy, hydroxyl, carboxyl, Cl, F, Br, I, amino, C₁ - C₆ mono or dialkyl amino, -NO₂, lower alkyl thio, -OCF₃, cyano, acylamino, -CF₃, trifluoroacetyl (i.e. 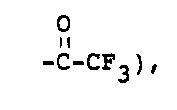
aminocarbonyl (i.e. 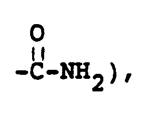 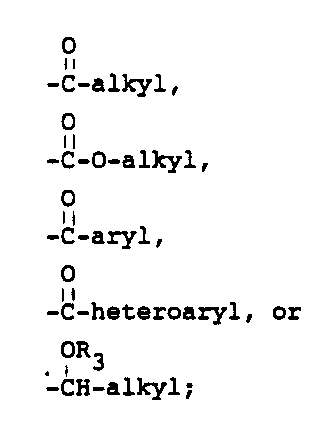
alkyl is lower alkyl;
aryl is phenyl or 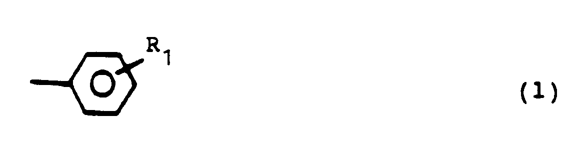
where R₁ is hydrogen, lower alkyl, C₁ - C₆ alkoxy, hydroxy, Cl, F, Br, I, C₁ - C₆ alkyl amino, -NO₂, -CN, -CF₃, -OCF₃;
heteroaryl is 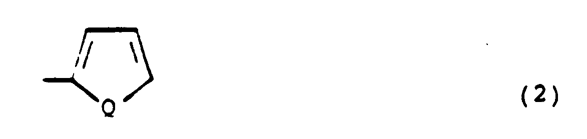
Q is -O-, -S-, 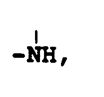
-CH=N;
R₃ is hydrogen, lower alkyl, or acyl; and
m is 1, 2, or 3.
When the compounds of the invention contain two or three R-substituents, each of the R-substituents can be independently selected from the above substituents. Preferably, each of the R-substituents is selected from the group consisting of hydrogen, C₁ - C₃ alkyl, C₁ - C₃ alkoxy, hydroxy, acyl, alkanoyl, Cl, F, Br, I, C₁ - C₃ alkyl amino, -NO₂, -CF₃, -OCF₃,

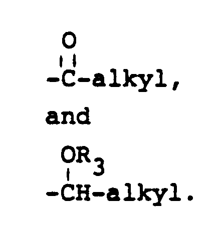

The compounds of the present invention are prepared in the following manner. The substituents R, R₁, R², R₃, X, Y, and Z and the integers m, n, and p are as defined above unless indicated otherwise.

### B. PREPARATION OF COMPOUNDS OF THE INVENTION

The compounds of the invention can be prepared by reacting a piperidine or a piperazine of the formula: 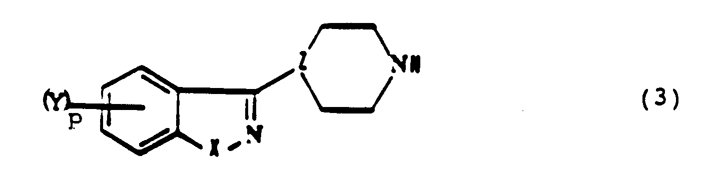
under alkylating conditions with a compound of the formula: 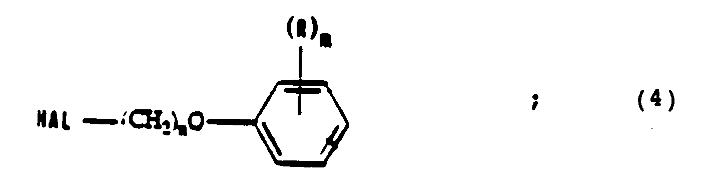
where HAL is Cl, Br, or I. The procedures that can be employed for preparing the piperidines, the piperazines, and the alkylating agents identified by the above formulas will now be described in detail.

### 1. Preparation of 3-(1-unsubstituted-4-piperazinyl)-1H-indazoles

Compounds of the formulae: 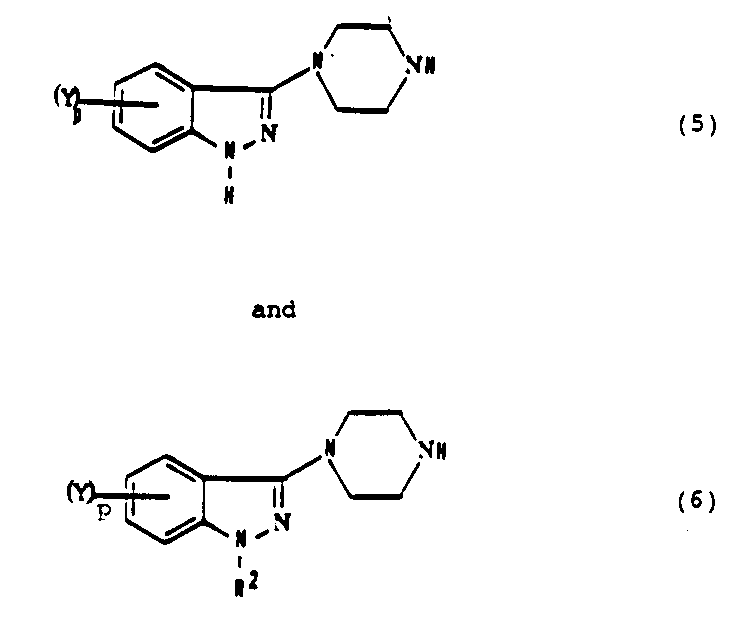
for use in synthesizing the indazoyl-substituted piperazines of the invention can be prepared as follows.

A substituted aryl ester of formula (7) is selected, 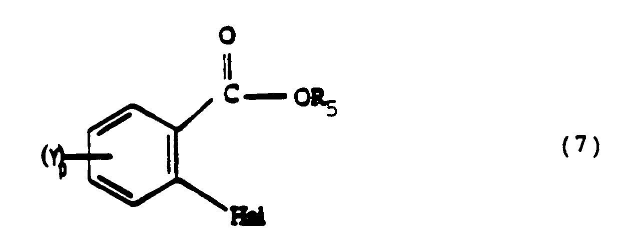
where R₅ is lower alkyl and Hal is a halogen selected from the group consisting of Cl, Br, and I. The ester of formula (7) is reacted with hydrazine, H₂NNH₂, under standard hydrazide formation conditions. Typically, the reaction is carried out in a nonreactive solvent, e.g. ethanol, methanol, or toluene, at a temperature of ambient temperature to the reflux temperature of the solvent for 4 to 16 hours to form a hydrazide of formula (8): 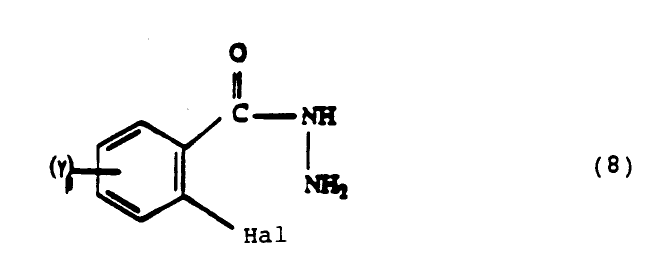
The hydrazide of formula (8) is reacted with a phenyl sulfonyl halide of the formula 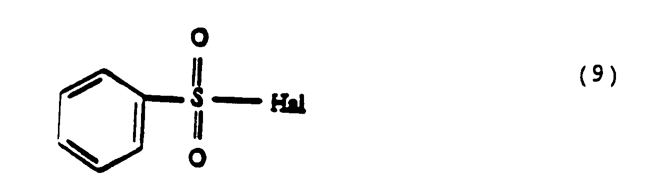
where Hal is a halogen selected from the group consisting of Cl and Br, to form a compound of the formula 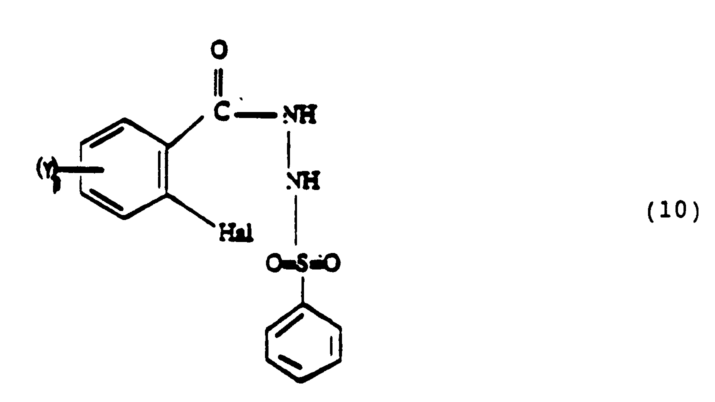
Typically this reaction is carried out in a basic solvent, such as pyridine or collidine, at a temperature of 0° to 30°C for 2 to 16 hours.

The compound of formula (10) in turn is reacted neat with thionyl chloride at a temperature of 50° to 79°C (reflux temperature) for 2 to 16 hours to form a compound of formula (11) 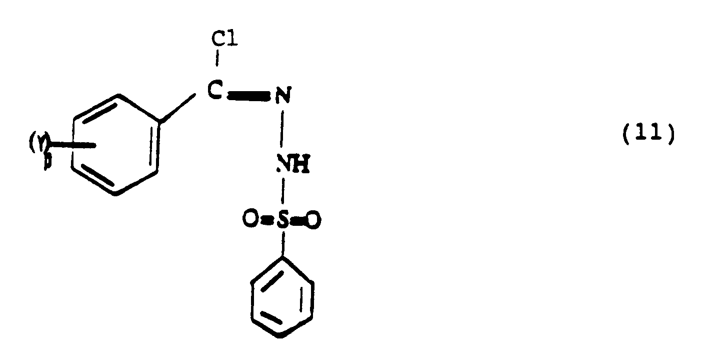
Compound (11) is reacted with a compound of formula (12), 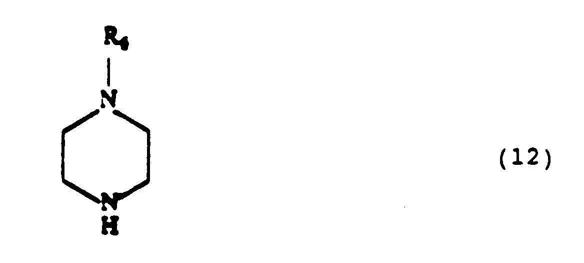
where R₆ is lower alkyl, under conventional nucleophilic reaction conditions, for example in an inert solvent, such as tetrahydrofuran (THF), toluene, or diethylether, at a temperature of 5° to 50°C for 1 to 16 hours to form a compound having the formula 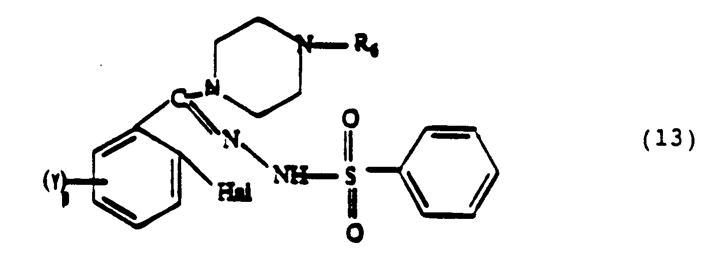
The compound of formula (13) is then reacted with a condensation agent, such as copper, copper-bronze, or cuprous oxide, in a solvent such as dimethylformamide, dimethyl acetamide, or tetramethyl urea, at a temperature of 120° to 177°C for 1 to 16 hours to form a piperazine-substituted phenylsulfonyl indazole of the formula 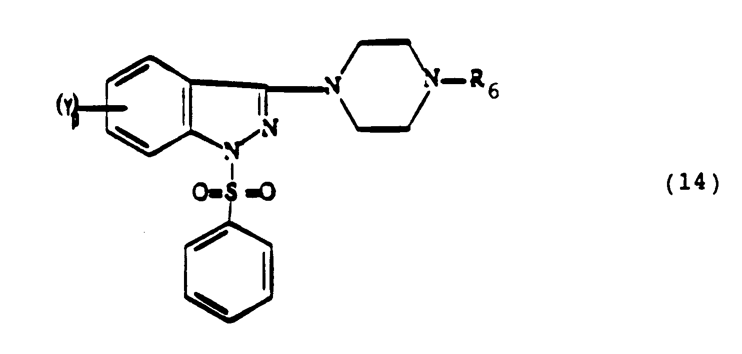
A cyano-substituted piperazine phenylsulfonyl indazole is then formed by reacting the compound of formula (14) with a conventional cyanation source, such as a halo-cyanide, e.g. BrCN or ClCN, under conventional cyanation conditions, typically in an inert solvent, e.g. dimethylsulfoxide (DMSO) or CHCl₃, at ambient temperature for 2 to 16 hours to form a compound of formula 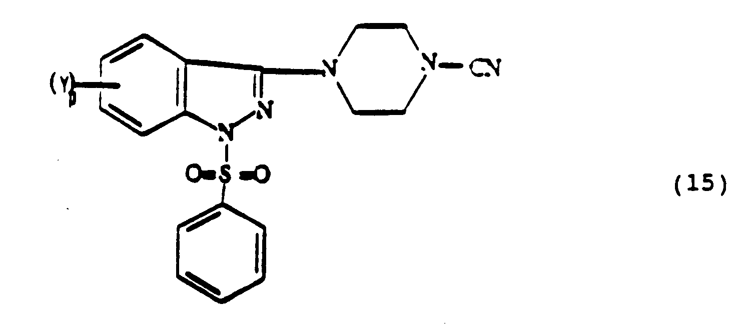
The compound of formula (15) is then subjected to reduction by means of a metal hydride, e.g. lithium aluminum hydride (LiAlH₄). Typically the reduction is carried out under standard reduction conditions in a solvent, such as tetrahydrofuran or diethyl ether, at a temperature of 35° to 67°C for 6 to 16 hours to form a compound of formula (16): 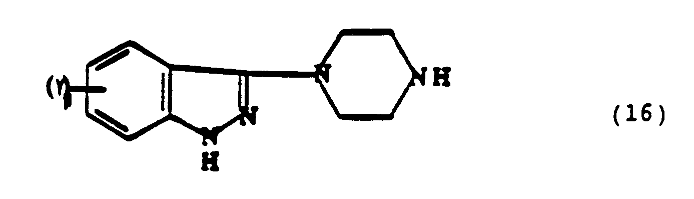
A compound of formula (16) can be formed in an alternative manner by first reacting a compound of formula (14) with a strong base, such as a metal alcoholate, e.g. sodium methoxide, sodium ethoxide, or sodium butoxide, or with KOH in tetrahydrofuran to form a compound of formula (17): 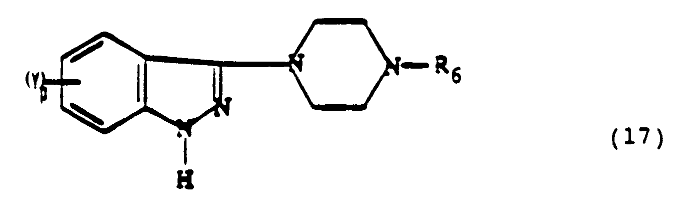
This reaction is typically carried out in a polar solvent, such as for example CH₃OH or C₂H₅OH, at a temperature of ambient to 50°C for 1 to 16 hours.

Alternatively, the compound of formula (17) can be formed by reducing compound (14) with LiAlH₄ under conditions as previously described.

The compound of formula (17) in turn can be reacted with a cyanation reagent, as previously described, to form a cyano substituted piperazine indazole of the formula 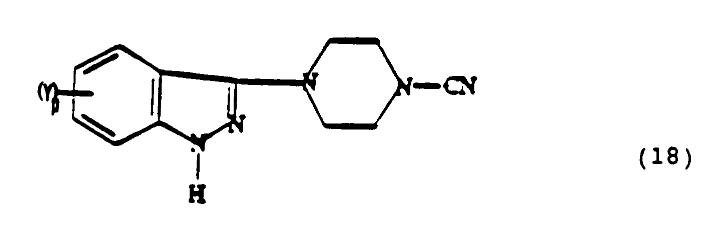
which in turn can be reduced with a metal hydride, as previously described, to form a compound of formula (16).

In an alternative embodiment, a compound of formula (18) can be reacted with an aqueous mineral acid, e.g. H₂SO₄ or HCl, at a temperature of 50° to 120°C for 2 to 16 hours to form a compound of formula (16).

### 2. Preparation of 3-(1-unsubstituted-4-piperazinyl)-1,2-benzisoxazoles

A compound of the formula: 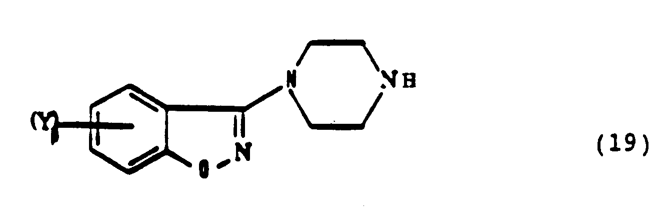
can be prepared according to conventional techniques. Suitable procedures are described in J. Med. Chem. 1986, 29:359. Compounds of formula (19) are useful for synthesizing the benzisoxazole-substituted piperazines of the invention.

### 3. Preparation of 3-(1-unsubstituted-4-piperazinyl)-1,2-benzisothiazoles

A compound of the formula: 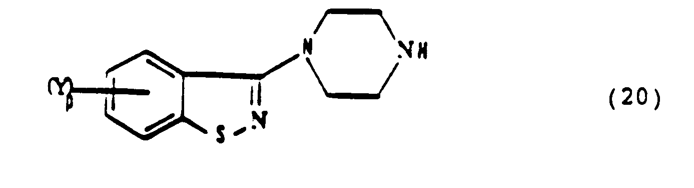
for use in synthesizing the benzisothiazole-substituted piperazines of the invention can be prepared according to the techniques described in J. Med. Chem. 1986, 29:359 and United Kingdom Patent (GB) 2 163 432 A.

### 4. Preparation of 3-(1-unsubstituted-4-piperidinyl)-1H-indazoles

A compound of the formula: 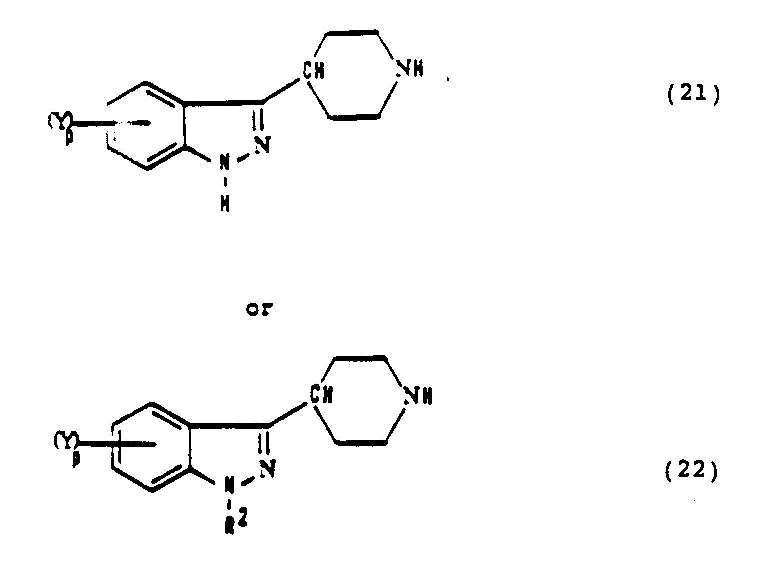
for use in synthesizing the indazole-substituted piperidines of the invention can be prepared using known techniques. For example, suitable techniques are described in substantial detail in U.S. Patent 4,710,573.

### 5. Preparation of 3-(1-unsubstituted-4-piperidinyl)-1,2-benzisoxazoles

A compound of the formula: 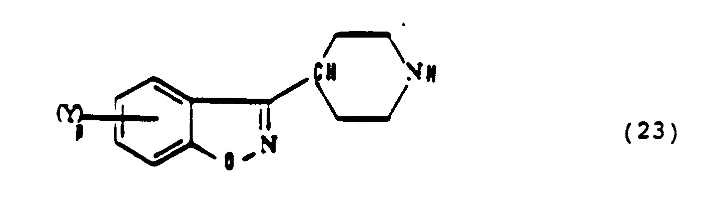
can be prepared by following the teachings from several sources. For example, U.S. Patent No. 4,355,037 contains a detailed description of compounds of formula (23) and of methods for preparing the compounds. Additional disclosure of methods for preparing the compounds of formula (23) can be found in U.S. Patent No. 4,327,103 and in Strupczewski et al., J. Med. Chem., 28:761-769 (1985). The compounds of formula (23) can be employed in the synthesis of the benzisoxazole substituted piperidines of the invention.

### 6. Preparation of 3-(1-unsubstituted-4-piperidinyl)-1,2-benzisothiazoles

Certain 3-(4-piperidinyl)-1,2-benzisothiazoles can be employed in the synthesis of the N-(aryloxyalkyl)heteroaryl piperidines of the invention. Specifically, a benzisothiazole of the formula: 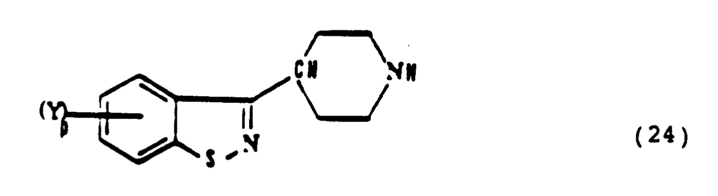
can be reacted with the alkylating agent previously described to form the N-(aryloxyalkyl)heteroarylpiperidines of the invention. Compounds of formula (24) and their methods of preparation are described in detail in U.S. patent 4,458,076.

### 7. Preparation of alkylating agents

The compounds described in Sections 1-6 above can be reacted with alkylating agents of the formula: 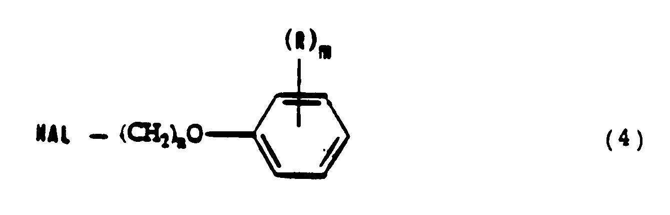
to form the N-(aryloxyalkyl)heteroarylpiperidines and piperazines of the invention. The alkylating agents of formula (4) and methods for preparing the alkylating agents are described in U.S. Patent No. 4,366,162. Additional disclosure can be found in South African publication ZA 86 14522.

### 8. Alkylation of heteroarylpiperidines and piperazines to form the compounds of the invention

The heteroarylpiperidines and piperazines described in Sections 1-6 above can be reacted under alkylating conditions with the alkylating agents described in Section 7 to form the compounds of this invention. The reaction can be carried out by dissolving the reagents in an inert solvent, such as dimethylformamide, acetonitrile, or butanol, and allowing the reagents to react from a temperature of 50°C to refluxing of the solvent in the presence of an acid receptor, such as a base. Examples of suitable bases are alkali metal carbonates, such as potassium carbonate, sodium carbonate, or sodium bicarbonate. The reaction can be carried out with or without a catalytic amount of an alkaline iodide, such as potassium iodide or sodium iodide, for a time sufficient to form a compound of formula (I) of the invention. Generally, the alkylation reaction is carried out for about 4 to about 16 hours, depending on reactivity of the reagents. The reaction temperature can vary from about 50° to about 120°C. The products can be isolated by treating the reaction product with water, extracting the product into an organic solvent that is immiscible in water, washing, drying, and concentrating the organic solvent to yield the free base, and then, if indicated, converting the resulting compound to an acid addition salt in a conventional manner.

Following are typical examples of compounds of the invention that can be prepared by following the techniques described above:
1-[4-[-3-[4-(1H-indazol-3-yl)-1-piperazinyl]propoxy]-3-methoxy phenyl]ethanone,
1-[4-[3-[4-(1,2-benzisoxazol-3-yl)-1-piperidinyl]propoxy]-3-methoxyphenyl]ethanone,
1-[4-[3-[4-(6-fluoro-1,2-benzisoxazol-3-yl)-1-piperidinyl]propoxy]-3-methoxyphenyl]ethanone,
1-[4-[4-[4-(1,2-benzisoxazol-3-yl)-1-piperidinyl]butoxy]-3-methoxyphenyl]ethanone,
1-[4-[4-[4-(6-fluoro-1,2-benzisoxazol-3-yl)-1-piperidinyl]butoxy]-3-methoxyphenyl]ethanone,
1-[4-[2-[4-(1,2-benzisoxazol-3-yl)-1-piperidinyl]ethoxy]-3-methoxyphenyl]ethanone fumarate,
1-[4-[4-[4-(1H-indazol-3-yl)-1-piperazinyl]butoxy]-3-methoxyphenyl]ethanone fumarate,
1-[4-[2-[4-(6-fluoro-1,2-benzisoxazol-3-yl)-1-piperidinyl]ethoxy]-3-methoxyphenyl]ethanone,
4-[3-[4-(6-fluoro-1,2-benzisoxazol-3-yl)-1-piperidinyl]propoxy]-3-methoxy-α-methylbenzenemethanol,
1-[4-[3-[4-(1,2-benzisothiazol-3-yl-1-piperidinyl]propoxy]-3-methoxyphenyl]ethanone,
1-[4-[3-[4-(6-fluoro-1,2-benzisoxazol-3-yl)-1-piperidinyl]propoxy]-3-hydroxyphenyl]ethanone,
1-[4-[3-[4-(6-fluoro-1H-indazol-3-yl)-1-piperazinyl]propoxy]-3-methoxyphenyl]ethanone,
1-[4-[4-[4-(6-fluoro-1H-indazol-3-yl)-1-piperazinyl]butoxy]-3-methoxyphenyl]ethanone,
1-[4-[3-[4-(1H-indazol-3-yl)-1-piperidinyl]propoxy]-3-methoxyphenyl]ethanone,
1-[4-[3-[4-(6-chloro-1,2-benzisoxazol-3-yl)-1-piperidinyl]propoxy]-3-methoxyphenyl]ethanone,
1-[4-[4-[4-(6-chloro-1,2-benzisoxazol-3-yl)-1-piperidinyl]butoxy]-3-methoxyphenyl]ethanone fumarate,
1-[4-[3-[4-[(5-fluoro-1,2-benzisoxazol-3-yl)-1-piperidinyl]propoxy]-3-methoxyphenyl]ethanone,
6-fluoro-3-[1-[3-(2-methoxyphenoxy)propyl]-4-piperidinyl]-1,2-benzisoxazole fumarate,
[4-[3-[4-(6-fluoro-1,2-benzoisoxazol-3-yl)-1-piperidinyl)propoxy]-3-methoxyphenyl]phenylmethanone,
1-[4-[4-[4-(1H-indazol-3-yl)-1-piperdinyl]butoxyl-3-methoxyphenyl]ethanone,
1-[4-[2-[4-(6-chloro-1,2-benzisoxazol-3-yl)-1-piperdinyl]ethoxy]-3-methoxyphenyl]ethanone,
1-[3-[3-[4-(6-fluoro-1,2-benzisoxazol-3-yl)-1-piperidinyl]propoxy]phenyl]ethanone fumarate,
1-[4-[3-[4-(6-fluoro-1,2-benzisoxazol-3-yl)-1-piperidinyl]propoxy]-2-methylphenyl]ethanone,
1-[2-[3-[4-(6-fluoro-1,2-benzisoxazol-3-yl)-1-piperidinyl]propoxy]-5-methylphenyl]ethanone,
N-[4-[3-[4-(6-fluoro-1,2-benzisoxazol-3-yl)-1-piperidinyl]propoxy]-3-methoxyphenyl]acetamide hemifumarate,
6-chloro-3-(1-piperazinyl)-1H-indazole,
1-[4-[3-[4-(6-fluoro-1H-indazol-3-yl)-1-piperidinyl]propoxy]-3-methoxyphenyl]ethanone,
1-[4-[3-[4-(6-fluoro-1,2-benzisoxazol-3-yl)-1-piperidinyl]propoxy]-3-methylphenyl]ethanone hemifumarate,
1-[4-[3-[4-(6-fluoro-1,2-benzisoxazol-3-yl)-1-piperidinyl]propoxy]phenyl]ethanone,
1-[4-[3-[4-(6-chloro-1H-indazol-3-yl)-1-piperazinyl]propoxy]-3-methoxyphenyl]ethanone,
1-[4-[4-[4-(1,2-benzisothiazol-3-yl)-1-piperazinyl]butoxy]-3-methoxyphenyl]ethanone,
4-[3-[4-(6-fluoro-1,2-benzisoxazol-3-yl)-1-piperidinyl]propoxy]-3-methoxybenzonitrile,
1-[4-[4-[4-(6-fluoro-1H-indazol-3-yl)-1-piperidinyl]butoxy]-3-methoxyphenyl]ethanone,
1-[4-[3-[4-(1-benzoyl-6-fluoro-1H-indazol-3-yl)-1-piperazinyl]propoxy]-3-methoxyphenyl]ethanone sesquifumarate,
1-[4-[4-[4-(6-chloro-1H-indazol-3-yl)-1-piperazinyl]butoxy]-3-methoxyphenyl]ethanone,
1-[4-[3-[4-(1,2-benzisothiazol-3-yl)-1-piperazinyl]propoxy]3-methoxyphenyl]ethanone hemifumarate,
1-[3,5-dibromo-4-[3-[4-(6-fluoro-1,2-benzisoxazol-3-yl)-1-piperidinyl]propoxy]phenyl]ethanone,
1-[4-[2-[4-(1,2-benzisothiazol-3-yl)-1-pipernzinyl]ethoxy]-3-methoxyphenyl]ethanone,
6-fluoro-3-[1-(3-phenoxypropyl)-4-piperidinyl]-1,2-benzisoxazole,
1-[4-[2-[4-(6-chloro-1H-indazol-3-yl)-1-piperazinyl]ethoxy]3-methoxyphenyl]ethanone,
1-[4-[3-[4-(6-fluoro-1,2-benzoisoxazol-3-yl)-piperidinyl]propoxy]-3-methylmercaptophenyl]ethanone,
1-[4-[4-[4-(1,2-benzisothiazol-3-yl)-1-piperidinyl]butoxy]-3-methoxyphenyl]ethanone,
1-[4-[3-[4-(6-fluoro-1,2-benzisoxazol-3-yl)-1-piperidinyl]propoxy]-3-methoxyphenyl]phenylmethanone,
1-[3-bromo-4-[3-[4-(6-fluoro-1,2-benzisoxazol-3-yl)-1-piperidinyl]propoxy]phenyl]ethanone,
3-[1-[3-[4-(1-ethoxyethyl)-2-methoxyphenoxy]propyl]-4-piperidinyl]-6-fluoro-1,2-benzisoxazole hydrocloride
3-[1-[3-[4-(1-acetoxyethyl)-2-methoxyphenoxy]propyl]-4-piperidinyl]-6-fluoro-1,2-benzisoxazole fumarate,
1-[4-[3-[4-(6-fluoro-1,2-benzisoxazol-3-yl]-1-piperidinyl]propoxy-3-methoxyphenyl]pentanone,
2-[3-[4-(6-fluoro-1,2-benzisoxazol-3-yl)-1-piperdinyl]-propoxy]-N-methylbenzenamine hemifumarate,
3-(1-[3-(4-bromo-2-methoxphenoxy)propyl]-4-piperidinyl)-6-fluoro-1,2-benzisoxazole,
1-[4-[3-[4-(6-fluoro-1,2-benzisoxazol-3-yl)-1-piperidinyl]propoxy]-3-methoxyphenyl]propanone,
4-[3-[4-(6-fluoro-1,2-benzisoxazol-3-yl)-1-piperidinyl]propoxy]-3-methoxybenzamide,
1-[4-[3-[4-(6-fluoro-1,2-benzisoxazol-3-yl)-1-piperidinyl]propoxy]-3-(methylamino)phenyl]ethanone, and
1-[4-[3-[4-(6-fluoro-1,2-benzisoxazol-3-yl)-1-piperidinyl]propoxy]-3-ethoxyphenyl]ethanone.

The compounds of the present invention are useful for treating psychoses by virtue of their ability to elicit an antipsychotic response in mammals. Antipsychotic activity is determined in the climbing mice assay by a method similar to those described by P. Protais, et al., Psychopharmacol., 50:1 (1976) and B. Costall, Eur. J. Pharmacol., 50:39 (1978).

Subject CK-1 male mice (23-27 grams) are group-housed under standard laboratory conditions. The mice are individually placed in wire mesh stick cages (4" X 10") and are allowed one hour for adaption and exploration of the new environment. Then apomorphine is injected subcutaneously at 1.5 mg/kg, a dose causing climbing in all subjects for 30 minutes. Compounds to be tested for antipsychotic activity are injected intraperitoneally or given oral doses at various time intervals, e.g. 30 minutes, 60 minutes, etc. prior to the apomorphine challenge at a screening dose of 10-60 mg/kg.

For evaluation of climbing, 3 readings are taken at 10, 20, and 30 minutes after apomorphine administration according to the following scale:

| Climbing Behavior Mice with: | Score |
|---|---|
| 4 paws on bottom (no climbing) | 0 |
| 2 paws on the wall (rearing) | 1 |
| 4 paws on the wall (full climb) | 2 |

Mice consistently climbing before the injection of apomorphine are discarded.

With full-developed apomorphine climbing, the animals are hanging on to the cage walls, rather motionless, over longer periods of time. By contrast, climbs due to mere motor stimulation usually only last a few seconds.

The climbing scores are individually totaled (maximal score: 6 per mouse over 3 readings) and the total score of the control group (vehicle intraperitoneally-apomorphine subcutaneously) is set to 100%. ED₅₀ values with 95% confidence limits, calculated by a linear regression analysis, of some of the compounds of the present invention as well as a standard antipsychotic agent are presented in Table 1.

**TABLE 1**

| COMPOUND | CLIMBING MOUSE ASSAY (ED₅₀ mg/kg, ip) |
|---|---|
| 1-[4-[3-[4-(1H-indazol-3-yl)-1-piperazinyl]propoxy]-3-methoxyphenyl]ethanone | 0.98 |
| 1-[4-[3-[4-(1,2-benzisoxazol-3-yl)-1-piperidinyl]propoxy]-3-methoxyphenyl]ethanone | 0.67 |
| 1-[4-[3-[4-(6-fluoro-1,2-benzisoxazol-3-yl)-1-piperidinyl]propoxy]-3-methoxyphenyl]ethanone | 0.095 |
| 1-[4-[4-[4-(1,2-benzisoxazol-3-yl)-1-piperidinyl]butoxy]-3-methoxyphenyl]ethanone | 1.6 |
| 1-[4-[4-[4-(6-fluoro-1,2-benzisoxazol-3-yl)-1-piperidinyl]butoxy]-3-methoxyphenyl]ethanone | 0.68 |
| Chlorpromazine (standard) | 1.3 |

Antipsychotic response is achieved when the compounds of the present invention are administered to a subject requiring such treatment as an effective oral, parenteral, or intravenous dose of from 0.01 to 50 mg/kg of body weight per day. It is to be understood, however, that for any particular subject, specific dosage regimens should be adjusted according to the individual need and the professional judgment of the person administering or supervising the administration of the aforesaid compound. It is to be further understood that the dosages set forth herein are exemplary only and they do not, to any extent, limit the scope or practice of the invention.

Some of the compounds of the present invention are also useful as analgetics due to their ability to alleviate pain in mammals. The analgetic utility is demonstrated in the phenyl p-quinone writhing assay in mice, a standard assay for analgesia: Proc. Soc. Exptl. Biol. Med., 95:729 (1957). Thus, for instance, the subcutaneous dose effecting an approximately 50% inhibition of writhing (ED₅₀) in mice produced in this assay is as shown in Table 2.

**TABLE 2**

| COMPOUND | INHIBITION OF PHENYLQUINONE INDUCED WRITHING ED₅₀mg/kg, sc |
|---|---|
| 1-[4-[3-[4-(1H-indazol-3-yl)-1-piperazinyl]propoxy]-3-methoxyphenyl]ethanone | 0.06 |
| 1-[4-[3-[4-(1,2-benzisoxazol-3-yl)-1-piperidinyl]propoxy]-3-methoxyphenyl]ethanone | 0.17 |
| 1-[4-[3-[4-(6-fluoro-1,2-benzisoxazol-3-yl)-1-piperidinyl]propoxy]-3-methoxyphenyl]ethanone | 0.03 |
| Propoxyphene (standard) | 3.9 |
| Pentazocine (standard) | 1.3 |

Analgesia is achieved when the compounds of the present invention are administered to a subject requiring such treatment as an effective oral, parenteral, or intravenous dose of from 0.01 to 100 mg/kg of body weight per day. It is to be understood, however, that for any particular subject, specific dosage regimens should be adjusted according to the individual need and the professional judgment of the person administering or supervising the administration of the aforesaid compound. It is to be further understood that the dosages set forth herein are exemplary only and that they do not, to any extent, limit the scope or practice of the invention.

Effective amounts of the compounds of the present invention can be administered to a subject by any one of several methods, for example, orally as in capsules or tablets, parenterally in the form of sterile solutions or suspensions, and in some cases intravenously in the form of sterile solutions.

The compounds of the present invention, while effective themselves, can be formulated and administered in the form of their pharmaceutically acceptable addition salts for purposes of stability, convenience of crystallization, increased solubility, and the like. Preferred pharmaceutically acceptable addition salts include salts of mineral acids, for example, hydrochloric acid, sulfuric acid, nitric acid, and the like; salts of monobasic carboxylic acids, for example, acetic acid, propionic acid, and the like; salts of dibasic carboxylic acids, for example, maleic acid, fumaric acid, and the like; and salts of tribasic carboxylic acids, such as carboxysuccinic acid, citric acid, and the like.

Effective quantities of the compounds of the invention can be administered orally, for example, with an inert diluent or with an edible carrier. They can be enclosed in gelatin capsules or compressed into tablets. For the purposes of oral therapeutic administration, compounds of the invention can be incorporated with an excipient and used in the form of tablets, troches, capsules, elixirs, suspensions, syrups, wafers, chewing gums, and the like. These preparations should contain at least 0.5% of active compound of the invention, but can be varied depending upon the particular form and can conveniently be between 4% to about 70% of the weight of the unit. The amount of active compound in such a composition is such that a suitable dosage will be obtained. Preferred compositions and preparations according to the present invention are prepared so that an oral dosage unit form contains between 1.0-300 milligrams of the active compound of the invention.

Tablets, pills, capsules, troches, and the like can also contain the following ingredients: a binder, such as microcrystalline cellulose, gum tragacanth, or gelatin; an excipient, such as starch or lactose; a disintegrating agent such as alginic acid, Primogel, corn starch, and the like; a lubricant such as magnesium stearate or Sterotes; a glidant such as colloidal silicon dioxide; and a sweetening agent such as sucrose; or saccharin, or a flavoring agent, such as peppermint, methyl salicylate, or orange flavoring. When the dosage unit form is a capsule, it can contain, in addition to materials of the above type, a liquid carrier such as a fatty oil. Other dosage unit forms can contain various materials that modify the physical form of the dosage unit, for example, as coatings. Thus, tablets or pills can be coated with sugar, shellac, or other enteric coating agents. A syrup can contain, in addition to the active compounds, sucrose as a sweetening agent and certain preservatives, dyes, colorings, and flavors. Materials used in preparing these various compositions should be pharmaceutically pure and non-toxic in the amounts used.

For the purpose of parenteral therapeutic administration, the active compound of the invention can be incorporated into a solution or suspension. These preparations should contain at least 0.1% of active compound, but can be varied between 0.5 and about 50% of the weight thereof. The amount of active compounds in such compositions is such that a suitable dosage will be obtained. Preferred compositions and preparations according to the present invention are prepared so that a parenteral dosage unit contains between 0.5 to 100 milligrams of active compound.

Solutions or suspensions can also include the following components: a sterile diluent, such as water for injection, saline solution, fixed oils, polyethylene glycols, glycerine, propylene glycol, or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates, or phosphates, and agents for the adjustment of tonicity such as sodium chloride or dextrose. The parenteral preparation can be enclosed in ampules, disposable syringes, or multiple dose vials made of glass or plastic.

The following examples are for illustrative purposes only and are not to be construed as limiting the invention. All temperatures are given in degrees Centigrade (°C.) unless indicated otherwise.

### EXAMPLE 1

### Preparation of 1-[4-[3-[4-(1H-Indazol-3-yl)-1-piperazinyl]propoxy]-3-methoxyphenyl]ethanone.

### (A) Synthesis of 2-bromobenzoic acid 2-phenylsulfonylhydrazide

To a solution of 2-bromobenzoic acid hydrazide (132 g) in pyridine (1.2ℓ) cooled to about 10° with an ice bath, was added benzensulfonyl chloride (78.3 ml). After complete addition, the reaction was stirred at ambient temperature for four hours, and then poured into ice-hydrochloric acid to precipitate a yellow solid, 135 g. The material was recrystallized from isopropanol to yield 125 g of 2-bromobenzoic acid 2-phenylsulfonylhydrazide, m.p. = 154-156°C.

### (B) Synthesis of α-chloro-2-bromobenzaldehyde phenylsulfonylhydrazone

A mixture of 2-bromobenzoic acid phenylsulfonylhydrazide (125 g, 0.35 mol) and thionyl chloride (265 ml) was stirred and refluxed for 2 hours. After about 15 minutes of reflux, the solid went into solution. The reaction was permitted to cool, and then it was poured into hexane. The resultant white solid was collected to afford 124 g of α-chloro-2-bromobenzaldehyde phenylsulfonylhydrazone, m.p. = 120-122°C.

### (C) Synthesis of 1-[[(phenylsulfonyl)hydrazono]-(2-bromophenyl)methyl]-4-methylpiperazine

To a stirred solution, under nitrogen, of α-chloro-2-bromobenzaldehyde phenylsulfonylhydrazone (271.1 g; 0.72 mol) in tetrahydrofuran (tetrahydrofuran, 2 liters), was added dropwise N-methylpiperazine (159.7 g; 1.6 mol). The reaction was stirred at ambient temperature for three hours, and then permitted to stand at ambient temperature for 16 hours. The reaction was chilled in an ice bath, and then filtered to remove the piperazine hydrochloride that was formed. The filtrate was concentrated to yield a brown gum. The gum was triturated with hot acetonitrile, the mixture was cooled in an ice bath, and when cold, was filtered to remove unwanted side product. The filtrate was then concentrated to afford 392.9 g of a brown gum of crude 1-[[(phenylsulfonyl)hydrazono]-(2-bromophenyl)methyl]-4-methylpiperazine.

### (D) Synthesis of 3-(4-Methyl-1-piperazinyl)-1-phenylsulfonyl-1H-indazole

A mixture of 1-[[(phenylsulfonyl)hydrazono]-(2-bromo phenyl)methyl]-4-methylpiperazine (31.0 g, 0.08 mol), copper bronze (3.1 g), K₂CO₃ (11.5 g), and dimethylformamide (500 ml), was stirred and refluxed for 1.5 hours. The reaction was poured into water and the aqueous suspension was stirred vigorously with ethyl acetate. The biphasic mixture was filtered through celite, and subsequently the layers were separated. The aqueous portion was extracted with another portion of ethyl acetate, and the combined extracts were washed (H₂O) and dried (MgSO₄). Concentration of the extract afforded a solid, which upon trituration with ether gave 19.7 g of solid. The solid was recrystallized from isopropanol to afford 17.7 g (60%) of product, m.p. 158-161°C. An analytical sample was obtained by another recrystallization from isopropanol (with charcoal treatment) to afford colorless crystals of the indazole, 3-(4-methyl-1-piperazinyl)-1-phenylsulfonyl-1H-indazole, m.p. = 160-161°C.

| ANALYSIS: | | | |
|---|---|---|---|
| Calculated for C₁₈H₂₀N₄O₂S: | 60.66%C | 5.66%H | 15.72%N |
| Found: | 60.45%C | 5.62%H | 15.61%N |

### (E) Synthesis of 4-[1-(Phenylsulfonyl)-1H-indazol-3-yl]-1-piperazinecarbonitrile

To a stirred mixture of 3-(4-methyl-1-piperazinyl)-1-phenylsulfonyl-1H-indazole (237 g, 0.67 mol), K₂CO₃ (102 g, 0.74 mol) and dimethylsulfoxide (DMSO, 2000 ml), under nitrogen, was added cyanogen bromide (72 g, 0.68 mol) dissolved in DMSO (525 ml). The reaction was stirred at ambient temperature for 5.5 hours and was then poured into H₂O (7 l). The solid, which precipitated from solution, was collected by filtration and was washed well with H₂O affording 168 g (68%) of product. A 5.2 g sample was recrystallized twice from ethanol-H₂O yielding 4.0 g of 4-[1-(phenylsulfonyl)-1H-indazol-3-yl]-1-piperazinecarbonitrile, m.p. = 178-180°C.

| ANALYSIS: | | | |
|---|---|---|---|
| Calculated for C₁₈H₁₇N₅O₂S: | 58.85%C | 4.66%H | 19.06%N |
| Found: | 59.01%C | 4.63%H | 19.09%N |

### (F) Synthesis of 3-(1-Piperazinyl)-1H-indazole

To a stirred mixture of 4-[1-(phenylsulfonyl)-1H-indazol-3-yl[-1-piperazinecarbonitrile (163 g, 0.44 mol) in tetrahydrofuran (2.0 l) was added, dropwise, lithium aluminum hydride (880 ml; 0.88 mol of a 1 M lithium aluminum hydride solution in tetrahydrofuran). After complete addition, the reaction was heated to reflux and stirred for 6 hours, stirred at ambient temperature for one hour and allowed to sit at room temperature overnight. The reaction was quenched by the careful dropwise addition of water. After no more hydrogen could be observed to evolve, the reaction was filtered and the lithium salt filter cake was washed well with tetrahydrofuran. The filtrate was combined with the filtrate of another run (all together the starting material totaled 300 g, i.e. 0.82 mol) and the combined filtrates were concentrated to afford 372 g of a yellow solid suspended in water. An attempt was made to partition the product between water and dichloromethane, but the product proved to be only slightly soluble in dichloromethane. Therefore, the biphasic product suspension was filtered through a course sintered funnel and the white product which was collected was dried to afford 121 g. The two phases of the filtrate were separated and the water was extracted again with dichloromethane. All of the dichloromethane phases were combined, washed twice with water, dried with magnesium sulfate, and concentrated to afford 41 g of a brown residue. The residue was triturated with diethyl ether and filtered to afford 10 g of a beige solid, m.p. = 139-150°C. The NMR and MS spectra of were consistent with the structure. Recrystallization of 10 g from toluene afforded 7.5 g of 3-(1-piperazinyl-1H-indazole, m.p. 153-155°C.

### (G) 3-(4-Methyl-1-piperazinyl)-1H-indazole

A stirred mixture of 3-(4-methyl-1-piperazinyl)-1-phenylsulfonyl-1H-indazole (13.5 g, 0.038 mol), methanol (150 ml) and 25% CH₃ONa in methanol (15.3 ml) was stirred and refluxed for 2.5 h. The reaction was concenrated to about one-tenth its volume, and water was added to the mixture, resulting in a red solution. The solution was extracted with dichloromethane, the extract washed (H₂O), dried (MgSO₄), and the solvent was concentrated to afford 6.6 g of a rose-colored solid. Two recrystallizations from toluene-hexane afforded 4.3 g (52%) of 3-(4-methyl-1-piperazinyl)-1H-indazole as an off-white solid, m.p. = 111-113°C.

| ANALYSIS: | | | |
|---|---|---|---|
| Calculated for C₁₂H₁₆N₄: | 66.64%C | 7.46%H | 25.91%N |
| Found: | 66.83%C | 7.42%H | 25.69%N |

### (H) 4-(1H-indazol-3-yl)-1-piperazinecarbonitrile

To a stirred mixture of cyanogen bromide (5.3 g, 0.05 mol), K₂CO₃ (7.1 g) and dimethylsulfoxide (40 ml) was added, dropwise, 3-(4-methyl-1-piperazinyl)-1H-indazole (11.0 g, 0.051 mol) dissolved in dimethylsulfoxide (60 ml). The reaction was stirred at ambient temperature for 1 h, and then it was poured into water. The aqueous suspension was extracted with ethyl acetate, the ethyl acetate was washed (H₂O), dried (MgSO₄), and concentrated to afford 7.8 g (67%) of a yellow solid. This sample was combined with another and recrystallized twice form toluene to afford analytically pure 4-(1H-indazol-3-yl)-1-piperazinecarbonitrile as a white solid, m.p. = 120-122°C.

| ANALYSIS: | | |
|---|---|---|
| Calculated for C₁₂H₁₃N₅: | 63.42%C | 5.76%H |
| Found: | 63.04%C | 5.84%H |

### (I) Synthesis of 3-(1-Piperazinyl)-1H-indazole

A mixture of 4-(1H-indazol-3-yl)-1-piperazinecarbonitrile (8.0 g, 0.04 mol) and 25% H₂SO₄ (100 ml) was stirred at reflux for 4.5 hours. The reaction was cooled in an ice bath and made basic by the dropwise addition of 50% NaOH. The basic solution was extracted with ethyl acetate. The ethyl acetate was washed with H₂O, dried with MgSO₄, and concentrated to afford 5.2 g (73%) of the desired compound, as a solid. The solid was recrystallized twice from toluene to afford 3.0 g of 3-(1-piperazinyl)-1H-indazole, m.p. = 153-155°C.

| ANALYSIS: | | | |
|---|---|---|---|
| Calculated for C₁₁H₁₄N₄: | 65.32%C | 6.98%H | 27.70%N |
| Found: | 65.21%C | 6.99%H | 27.80%N |

### (J) Synthesis of 1-[4-[3-[4-(1H-Indazol-3-yl)-1-piperazinyl]propoxy]-3-methoxyphenyl]ethanone

A mixture of 3-(1-piperazinyl)-1H-indazole (4.0g, 0.02 mol), K₂CO₃(3.0g, 0.022 mol), 1-[4-(3-chloropropoxy)-3-methoxyphenyl]ethanone (5.3g, 0.022 mol), a few crystals of KI, and dimethylformamide (60 ml) was stirred at 90°C for 5 hours. The reaction was poured into water, and the aqueous mixture was extracted with ethyl acetate. The extract was washed (brine), dried (MgSO₄), and the solvent was concentrated to afford a white solid, which was triturated with diethyl ether and collected to yield 7.0g of product. Two recrystallizations from absolute ethyl alcohol yielded 5.3g (64%) of analytically pure 1-[4-[3-[4-(1H-indazol-3-yl)-1-piperazinyl]propoxy]-3-methoxyphenyl]-ethanone, m.p. = 155-157°C.

| ANALYSIS: | | | |
|---|---|---|---|
| Calculated for C₂₃H₂₈N₄O₃: | 67.62%C | 6.91%H | 13.72%N |
| Found: | 67.45%C | 6.74%H | 13.56%N |

### EXAMPLE 2

### 1-[4-[3-[4-(1,2-Benzisoxazol-3-yl)-1-piperidinyl]propoxy]-3-methoxyphenyl]ethanone

A mixture of 3-(4-piperidinyl)-1,2-benzisoxazole hydrochloride (4.8g, 0.02 mol), K₂CO₃ (5.2g, 0.04 mol), 1-[4-(3-chloropropoxy)-3-methoxyphenyl]ethanone (5.3g, 0.022 mol), a few crystals of KI and dimethylformamide (60 ml) was stirred at 90°C for 16 hours. The reaction was poured into water and the aqueous mixture was extracted with ethyl acetate. The extract was washed (water), dried (MgSO₄) and concentrated to afford a brown oil. The oil was chromatographed on a ®Waters Prep 500 utilizing silica gel columns and ethyl acetate-diethylamine (2%), as eluent. Concentration of the appropriate fractions afforded 3.9 g of product as an off-white solid. Recrystallization from absolute ethyl alcohol afforded 2.6g (33%) of 1-[4-[3-[4-(1,2-benzisoxazol-3-yl)-1-piperidinyl]propoxy]-3-methoxyphenyl]ethanone, m.p. = 102-104°C, as colorless needles.

| ANALYSIS: | | | |
|---|---|---|---|
| Calculated for C₂₄H₂₈N₂O₄: | 70.56%C | 6.91%H | 6.86%N |
| Found: | 70.73%C | 6.93%H | 6.85%N |

### EXAMPLE 3

### 1-[4-[3-[4-(6-Fluoro-1,2-benzisoxazol-3-yl)-1-piperidinyl]propoxy]-3-methoxyphenyl]ethanone

A stirred mixture of 6-fluoro-3-(4-piperidinyl)-1,2 benzisoxazole hydrochloride (5.1g, 0.02 mol), K₂CO₃ (5.2g, 0.04 mol), 1-[4-(3-chloropropoxy)-3-methoxyphenyl]ethanone (5.3g, 0.022 mol), and dimethylformamide (60 ml) was heated at 90°C for 16 hours. The reaction was poured into water, and the aqueous mixture was extracted with ethyl acetate. The ethyl acetate was washed (water), dried (MgSO₄) and concentrated to afford a moist solid. Recrystallization (twice) from ethyl alcohol afforded 5.0g (58%) of 1-[4-[3-[4-(6-fluoro-1,2-benzisoxazol-3-yl)-1-piperidinyl]propoxy]3-methoxyphenyl]ethanone] as a beige solid, m.p. = 118-120°C.

| ANALYSIS: | | | |
|---|---|---|---|
| Calculated for C₂₄H₂₇FN₂O₄: | 67.60%C | 6.38%H | 6.57%N |
| Found: | 67.47%C | 6.40%H | 6.53%N. |

### EXAMPLE 4

### 1-[4-[4-[4-(1,2-Benzisoxazol-3-yl)-1-piperidinyl]butoxy]-3-methoxyphenyl]ethanone

A mixture of 3-(4-piperidinyl)-1,2-benzisoxazole hydrochloride (4.3g, 0.018 mol), K₂CO₃ (5.5g, 0.04 mol), and 1-[4-(4-bromobutoxy)-3-methoxyphenyl]ethanone (5.5g, 0.018 mol), and dimethylformamide (60 ml) was stirred and heated at 75°C for 16 hours. The reaction was poured into water and was extracted with ethyl acetate. The ethyl acetate was washed (water), dried (MgSO₄), and the solvent concentrated to afford 7.2g of a beige solid. Recrystallization (twice) from ethyl alcohol yielded 3.3g (43%) of 1-[4-[4-[4-(1,2-benzisoxazol-3-yl)-1-piperidinyl]butoxy]-3-methoxyphenyl]ethanone, m.p. = 99-101°C.

| ANALYSIS: | | | |
|---|---|---|---|
| Calculated for C₂₅H₃₀N₂O₄: | 71.11%C | 7.16%H | 6.63%N |
| Found: | 70.76%C | 7.24%H | 6.58%N. |

### EXAMPLE 5

### 1-[4-[4-(4-(6-Fluoro-1,2-benzisoxazol-3-yl)-1-piperidinyl]butoxy]-3-methoxyphenyl]ethanone

A stirred mixture of 6-fluoro-3-(4-piperidinyl)-1,2-benzisoxazole hydrochloride (5.1g, 0.02 mol), K₂CO₃ (5.2g, 0.04 mol), 1-[4-(4-bromobutoxy)-3-methoxyphenyl]ethanone (6.6g, 0.022 mol), and dimethylformamide (60 ml) was heated at 75°C for 5 hours. The reaction was poured into water, and the aqueous mixture was extracted with ethyl acetate. The ethyl acetate was washed (water), dried (MgSO₄), and the solvent was concentrated to yield initially an oil, which solidified upon standing. The solid was triturated with hexane and collected to afford 7.7g of the product as a waxy solid. The compound was chromatographed on a ®Waters Prep 500 utilizing silica gel columns and eluting with dichloromethane/methanol (5%). Concentration of the appropriate fractions yielded 5.1g of off-white solid 1-[4-[4-[4-(6-fluoro-1,2-benzisoxazol-3-yl)-1-piperidinyl]butoxy]-3-methoxyphenyl]ethanone, which when recrystallized from ethyl alcohol yielded 3.2g (36%) of feathery-white needles, m.p. = 88-90°C.

| ANALYSIS: | | | |
|---|---|---|---|
| Calculated for C₂₅H₂₉FN₂O₄: | 68.16%C | 6.64%H | 6.36%N |
| Found: | 67.96%C | 6.49%H | 6.29%N. |

### EXAMPLE 6

### 1-[4-[2-[4-(1,2-Benzisoxazol-3-yl)-1-piperidinyl]ethoxy]-3-methoxyphenyl]ethanone fumarate

A mixture of 3-(4-piperidinyl)-1,2-benzisoxazole hydrochloride (4.8g, 0.02 mol), K₂CO₃ (5.2g, 0.04 mol), 1-[4-(2-chloroethoxy)-3-methoxyphenyl]ethanone (5.0g, 0.022 mol), and dimethylformamide (90 ml) was heated at 90°C for 16 hours. The reaction was poured into water and the aqueous mixture was extracted with ethyl acetate. The ethyl acetate was washed (water), dried (MgSO₄), and the solvent was concentrated to afford an oil. Upon standing, the oil solidified to afford a beige solid. The crude solid was recrystallized twice from ethyl alcohol to afford 5.9 g of an off-white solid. The solid was dissolved in ethyl acetate, and fumaric acid (1.2g, 1.1 equiv.) was added. The mixture was heated briefly on a steam bath, and then stirred at ambient temperature for 2 hours. An initial green oil settled out and the supernatant solution was decanted. Ether was added to the decantate and 4.0g of a white fumarate salt was collected. The salt was recrystallized twice from ethanol-ether to yield 1.7g (17%) of 1-[4-[2-[4-(1,2-benzisoxazol-3-yl)-1-piperidinyl]ethoxy]-3-methoxyphenyl]ethanone fumarate, m.p. = 127-129°C.

| ANALYSIS: | | | |
|---|---|---|---|
| Calculated for C₂₃H₂₆N₂O₄ · C₄H₄O₄: | 63.52%C | 5.92%H | 5.49%N |
| Found: | 63.00%C | 5.87%H | 5.42%N |

### EXAMPLE 7

### 1-[4-[4-[4-(1H-Indazol-3-yl)-1-piperazinyl]butoxyl-3-methoxyphenyl]ethanone fumarate

A stirred mixture of 3-(1-piperazinyl)-1H-indazole (4.0 g, 0.02 mol), K₂CO₃ (3.0 g, 0.023 mol), 1-[4-(4-bromobutoxy)-3-methoxyphenyl]ethanone (5.3 g) and dimethylformamide (60 ml) was heated at 75°C for 6 hours.

The reaction was poured into water, and a white solid precipitated from solution. The solid was collected and dried to afford 7.2g of the crude product. The crude solid was recrystallized twice from ethyl alcohol to yield 4.1g of the free base, which was converted to its fumarate salt by the addition of fumaric acid (1.1g) to the compound dissolved in refluxing acetone. The resulting fumarate salt (5.0g) was recrystallized from ethyl alcohol to afford 3.8g (35%) of 1-[4-[4-[4-(1H-indazol-3-yl)-1-piperazinyl]butoxy]-3-methoxy phenyl]ethanone fumarate, as a white solid, m.p. = 163-165°C.

| ANALYSIS: | | | |
|---|---|---|---|
| Calculated for C₂₄H₃₀N₄O₃.C₄H₄O₄: | 62.44%C | 6.36%H | 10.40%N |
| Found: | 62.28%C | 6.62%H | 10.34%N. |

### EXAMPLE 8

### 1-(4-[2-[4-(6-Fluoro-1,2-benzisoxazol-3-yl)-1-piperidinyl]ethoxy]-3-methoxyphenyl]ethanone

A stirred mixture of 6-fluoro-3-(4-piperidinyl)-1,2 benzisoxazole hydrochloride (5.1g, 0.02 mol), K₂CO₃ (5.2g), 1-[4-(2-chloroethoxy)-3-methoxyphenyl]ethanone (5.0g, 1.022 mol), and dimethylformamide (90 ml) was heated at 90°C for 16 hours. The reaction was poured into water, and the aqueous mixture was extracted with ethyl acetate. The ethyl acetate was washed (water), dried (MgSO₄), and concentrated to afford 7.4g of a yellow solid. The solid was chromatographed on a ®Waters Prep LC 500 utilizing dichloromethane/methanol (4%) as eluent, and subsequent concentration of the appropriate fraction afforded 4.0g of a yellow solid. The solid was recrystallized from ethyl alcohol to yield 3.1g (38%) of 1-[4-[2-[4-(6-fluoro-1,2-benzisoxazol-3-yl)-1-piperidinyl]ethoxy]-3-methoxyphenyl]ethanone, as slightly yellow flakes, m.p. = 132-134°C.

| ANALYSIS: | | | |
|---|---|---|---|
| Calculated for C₂₃H₂₅FN₂O₄: | 66.98%C | 6.11%H | 6.79%N |
| Found: | 66.90%C | 6.20%H | 6.74%N. |

### EXAMPLE 9

### 4-[3-[4-(6-Fluoro-1,2-benzisoxazol-3-yl)-1-piperidinyl]propoxy]-3-methoxy-^a-methylbenzenemethanol

To a stirred mixture of 1-[4-[3-[4-(6-fluoro-1,2-benzisoxazol-3-yl)-1-piperidinyl]propoxy-3-methoxyphenyl]ethanone (4.0 g, 0.0094 mol) in methanol/tetrahydrofuran (60 ml, 1:1), was added sodium borohydride (0.4 g, 0.01 mol). After an initial evolution of gas, all insolubles went into solution. The reaction was stirred at ambient temperature for 3 hours and TLC at this time showed a very slight amount of starting ketone. Therefore, another 0.1 g of sodium borohydride was added, and stirring was continued for an additional 0.5 hour. TLC now showed complete disappearance of starting material. The reaction was concentrated to an off-white residue, which was diluted with water and collected to yield 3.4 g of alcohol. This was recrystallized from toluene (twice, with a charcoal treatment) to yield 2.7 g (67%) of 4-[3-[4-(6-fluoro-1,2-benzisoxazol-3-yl)-1-piperidinyl-]-3-methoxy-^a-methylbenzenemethanol as a white solid, m.p. = 136-138°C.

| ANALYSIS: | | | |
|---|---|---|---|
| Calculated for C₂₄H₂₉FN₂O₄: | 67.27%C | 6.82%H | 6.54%N |
| Found: | 67.59%C | 6.89%H | 6.47%N |

### EXAMPLE 10

### 1-[4-[3-[4-(1,2-Benzisothiazol-3-yl)-1-piperidinyl]propoxy]-3-methoxyphenyl]ethanone

A mixture of 3-(4-piperidinyl)-1,2-benzisothiazole (3.0 g, 0.0137 mol), potassium carbonate (2.3 g, 0.0165 mol), 1-[4-(3-chloropropoxy)-3-methoxyphenyl]ethanone (4.0 g, 0.0165 mol), potassium iodide (200 mg) and acetonitrile (100 ml) was stirred at reflux under N₂ for 24 hours. The cooled reaction was filtered and the cake was washed well with acetonitrile. The filtrate was concentrated to an oily residue, which was partitioned between water and ethyl acetate. The ethyl acetate extract was washed well with water, dried with MgSO₄ and concentrated to yield 6.1 g of a beige oil which solidified upon standing. The product was triturated with diethyl ether and filtered to give 4.2 g of a beige solid. The compound was recrystallized from ethyl alcohol to afford 3.5 g, and another recrystallization from ethyl alcohol (utilizing decolorizing carbon) provided 2.4 g (41%) of 1-[4-[3-[4-(1,2-benzisothiazol-3-yl)-1-piperidinyl]propoxy]-3-methoxyphenyl]ethanone, m.p. 93-95°C.

| ANALYSIS: | | | |
|---|---|---|---|
| Calculated for C₂₄H₂₈N₂O₃S: | 67.90%C | 6.65%H | 6.60%N |
| Found: | 67.89%C | 6.61%H | 6.59%N |

### EXAMPLE 11

### 1-(4-[3-[4-(6-Fluoro-1,2-benzisoxazol-3-yl)-1-piperidinyl]propoxy]-3-hydroxyphenyl]ethanone

### (A) Synthesis of 1-[4-(3-chloropropoxy)-3-hydroxyphenyl]ethanone

To a stirred solution of 1-[4-(3-chloropropoxy)-3 methoxyphenyl]ethanone (10.0 g, 0.041 mol) in methylene chloride (120 ml) cooled to -50°C (dry ice-methanol) was added, dropwise, 1M boron tribromide in methylene chloride (123 ml, 0.12 mol). The temperature was kept between -40°C and -50°C. After complete addition, the reaction was permitted to reach -30°C, and the TLC checked (ca. 15 min. after final boron tribromide was added). Saturated NaHCO₃ was added, dropwise, never allowing the temperature to go above 0°C during most of the addition. When sufficient NaHCO₃ had been added to make the solution basic, the organic layer was collected. The layer was washed with brine, dried (MgSO₄), and concentrated to yield 8.1 g of dark brown oil, which solidified on standing. This was chromatographed on a ®Waters Prep 500 LC (2 silica columns, 2% methanol-methylene chloride as eluent). Upon concentration of the appropriate fractions, 5.8 g of a brown tacky solid were obtained. This was recrystallized from isopropyl ether (with decanting of the yellow isopropyl ether supernatant from the dark brown oily residue) to give initially 2.5 g of a yellow solid. Concentration of the mother liquor gave an additional 0.5 g, m.p. = 110-113°C.

### (B) Synthesis of 1-[4-[3-[4-(6-Fluoro-1,2-benzisoxazol-3-yl)-1-piperidinyl]propoxy]-3-hydroxyphenyl]ethanone

A stirred mixture of 6-fluoro-3-(4-piperidinyl)-1,2-benzisoxazole (2.8 g, 0.013 mol), NaHCO₃ (1.1 g), several crystals of KI, 1-[4-(3-chloropropoxy)-3-hydroxyphenyl]ethanone, and acetonitrile (100 ml) was refluxed for 16 hours. The reaction was poured into water, and the aqueous mixture was extracted with ethyl acetate. The organic extract was washed (water), dried (MgSO₄), and the solvent was concentrated to afford 5.7 g of a thick yellow oil. The oil was chromatographed on a ®Waters Prep 500 LC on silica gel, eluting with 7% methanol/methylene chloride. Concentration of the appropriate fraction afforded a yellow oil, which upon standing yielded 3.5 g of the compound as a pale, yellow solid. The solid was recrystallized from ethyl alcohol to afford 2.7 g (50%) of 1-[4-[3-[4-(6-fluoro-1,2-benzisoxazol-3-yl)-1-piperidinyl]propoxy]-3-hydroxyphenyl]-ethanone as a pale yellow solid, m.p. = 122-124°C.

| ANALYSIS: | | | |
|---|---|---|---|
| Calculated for C₂₃H₂₅FN₂O₄: | 66.98%C | 6.11%H | 6.79%N |
| Found: | 66.97%C | 6.20%H | 6.69%N |

### EXAMPLE 12

### 1-[4-[3-[4-(6-Fluoro-1H-indazol-3-yl)-1-piperazinyl] propoxy]-3-methoxyphenyl]ethanone

A stirred mixture of 6-fluoro-3-(1-piperazinyl)-1H-indazole (2.3 g, 0.01 mol), K₂CO₃ (1.5 g), 1-[4-(3-chloropropoxy)-3-methoxyphenyl]ethanone (2.8 g, 0.011 mol), several crystals of KI and dimethylformamide (60 ml) was heated at 90°C for 16 hours. The reaction was poured into H₂O, and the aqueous suspension was extracted with ethyl acetate. The ethyl acetate was washed (H₂O), dried (MgSO₄) and concentrated to afford 5.0 g of a yellow oil. The oil was chromatographed on a ®Waters Prep 500 utilizing silica gel columns and eluting with methylene chloride/methanol (7%). Concentration of the desired fractions yielded 2.0 g (46%) of an off-white solid. This sample was combined with 1.0 g of a previous sample, and this was recrystallized from toluene to afford 2.6 g of 1-[4-[3-[4-(6-fluoro-1H-indazol-3-yl)-1-piperazinyl]propoxy]-3-methoxyphenyl]ethanone as a white solid, m.p. = 135-137°C.

| ANALYSIS: | | | |
|---|---|---|---|
| Calculated for C₂₃H₂₇FN₄O₃: | 64.77%C | 6.38%H | 13.14%N |
| Found: | 64.66%C | 6.21%H | 13.02%N |

### EXAMPLE 13

### 1-[4-[4-[4-(6-Fluoro-1H-indazol-3-yl)-1-piperazinyl] butoxy]-3-methoxyphenyl]ethanone

A stirred mixture of 6-fluoro-3-(1-piperazinyl)-1H-indazole hydrochloride (5.0 g, 0.019 ml), K₂CO₃ (5.8 g) and 1-[4-(4-bromobutoxy)-3-methoxyphenyl]ethanone (6.3 g, 0.021 mol) and dimethylformamide (80 ml) was heated at 75°C for 6 hours. The reaction was poured into water, and an off-white solid formed from solution. The solid was collected and dried to yield 4.5 g of crude product. The compound was recrystallized from ethanol (3 times) to afford 3.0 g of an off-white solid. The solid was chromatographed on a ®Waters Prep 500 utilizing silca gel columns and eluting with methylene chloride/methanol (7%). Concentration of the appropriate fractions afford 2.3 g of an off-white solid, which when recrystallized from ethanol yielded 1.9 g (26%) of analytically pure 1-[4-[4-[4-(6-fluoro-1H-indazol-3-yl)-1-piperazinyl]butoxy]-3-methoxyphenyl]ethanone, m.p. = 156-158°C.

| ANALYSIS: | | | |
|---|---|---|---|
| Calculated for C₂₄H₂₉FN₄O₃: | 65.44%C | 6.64%H | 12.72%N |
| Found: | 65.38%C | 6.49%H | 12.60%N |

### EXAMPLE 14

### 1-[4-[3-[4-(1H-Indazol-3-yl)-1-piperidinyl] propoxy]-3-methoxyphenyl]ethanone

A mixture of 3-(4-piperidinyl)-1H-indazole (3.0 g, 0.015 mol), K₂CO₃ (1.6 g), 1-[4-(3-chloropropoxy)-3-methoxy phenyl]ethanone (5.3 g, 0.022 mol), a few crystals of KI and acetonitrile (100 ml) was stirred and refluxed for 16 hours. The reaction was poured into water and a white solid separated from solution. The solid was collected, dried and afforded 5.1 g of product. Recrystallization from ethanol yielded 3.6 g of the compound, which upon chromatography (preparative HPLC on silica gel, eluting with methylene chloride/methanol-9:1) gave 3.0 g (49%) of an off-white solid. Recrystallization from ethanol afforded the analytically pure 1-[4-[3-[4-(1H-indazol-3-yl)-1-piperidinyl]propoxy]-3-methoxyphenyl]ethanone as a white solid, m.p. = 171-173°C.

| ANALYSIS: | | | |
|---|---|---|---|
| Calculated for C₂₄H₂₉N₃O₃: | 70.74%C | 7.17%H | 10.31%N |
| Found: | 70.52%C | 7.27%H | 10.42%N |

### EXAMPLE 15

### 1-[4-[3-[4-(6-Chloro-1,2-benzisoxazol-3-yl)-1-piperidinyl]propoxy]-3-methoxyphenyl]ethanone

A stirred mixture of 6-chloro-3-(4-piperidinyl)-1,2-benzisoxazole (4.7 g, 0.02 mol), 1-[4-(3-chloropropoxy)-3-methoxyphenyl]ethanone (4.8 g, 0.02 mol), K₂CO₃(2.8 g), several crystals of KI and acetonitrile (120 ml) was refluxed for 16 hours. The reaction was filtered and the filtrate was concentrated to yield a solid-oil mixture. The residue was chromatographed on a ®Waters Prep 500 utilizing silica columns and eluting with methylene chloride/methanol (5%). Concentration of the desired fractions yielded 3.2 g of a beige solid, which upon recrystallization from ethanol afforded 2.7 g (31%) of 1-[4-[3-[4-(6-chloro-1,2-benzisoxazol-3-yl)-1-piperidinyl]propoxy]-3-methoxyphenyl]ethanone as a beige solid; m.p. = 116-118°C.

| ANALYSIS: | | | |
|---|---|---|---|
| Calculated for C₂₄H₂₇ClN₂O₄: | 65.08%C | 6.14%H | 6.32%N |
| Found: | 65.35%C | 6.22%H | 6.28%N |

### EXAMPLE 16

### 1-[4-[4-[4-(6-Chloro-1,2-benzisoxazol-3-yl)-1-piperidinyl]butoxy]-3-methoxyphenyl]ethanone fumarate

A stirred mixture of 6-chloro-3-(4-piperidinyl)-1,2-benzisoxazole (4.7 g, 0.02 mol), 1-[4-(4-bromobutoxy)-3-methoxyphenyl]ethanone (6.0 g, 0.02 mol), K₂CO₃ (2.8 g) and acetonitrile (120 ml) was refluxed for 16 hours. The reaction was allowed to cool, filtered, and the filtrate was concentrated to 9.9 g of a brown oil. The oil was chromatographed on a ®Waters Prep 500 utilizing silica gel columns and eluting with methylene chloride/methanol (5%). Concentration of the appropriate fractions afforded 2.3 g of an off-white solid. The solid was dissolved in ethanol and was evaporated, and the resulting brown solid was taken up in refluxing acetone. Upon cooling, a white solid crystallized from solution yielding 2.2 g (19%) of 1-[4-[4-[4-(6-chloro-1,2-benzisoxazol-3-yl)-1-piperidinyl]butoxy]-3-methoxyphenyl]ethanone fumarate as a white solid, m.p. = 139-141°C.

| ANALYSIS: | | | |
|---|---|---|---|
| Calculated for C₂₅H₂₉ClN₂O₄·C₄H₄O₄: | 60.78%C | 5.80%H | 4.89%N |
| Found: | 60.69%C | 5.74%H | 4.85%N |

### EXAMPLE 17

### 1-[4-[3-[4-(5-Fluoro-1,2-benzisoxazol-3-yl)-1-piperidinyl]propoxy]-3-methoxyphenyl]ethanone

A mixture of 5-fluoro-3-(4-piperidinyl)-1,2-benzisoxazole (2.2 g, 0.01 mole), 1-[4-(3-chloropropoxy)-3-methoxyphenyl]-ethanone (2.4 g, 0.01 mole), K₂CO₃ (1.4 g), a few crystals of KI and acetonitrile (100 ml) was stirred and refluxed for 8 hours. The reaction was poured into water and the aqueous mixture was extracted with ethyl acetate. The ethyl acetate extract was washed (brine), dried (MgSO₄), and concentrated to afford 4.0 g of a white solid. The solid was chromatographed on a ®Waters Prep 500 HPLC utilizing silica gel columns and eluting with methylene chloride/methanol (5%). Concentration of the appropriate fractions afforded 2.0 g (47%) of 1-[4-[3-[4-(5-fluoro-1,2-benzisoxazol-3-yl)-1-piperidinyl]propoxy]-3-methoxyphenyl]ethanone as a white crystalline solid, m.p. = 103-105°C.

| ANALYSIS: | | | |
|---|---|---|---|
| Calculated for C₂₄H₂₇FN₂O₄: | 67.59%C | 6.38%H | 6.57%N |
| Found: | 67.50%C | 6.47%H | 6.53%N |

### EXAMPLE 18

### 6-Fluoro-3-[1-[3-(2-methoxyphenoxy)propyl]-4-piperidinyl]-1,2-benzisoxazole fumarate

A stirred mixture of 6-fluoro-3-(4-piperidinyl]-1,2-benzisoxazole (2.45 g; 11.1 mmoles), K₂CO₃ (2.0 g), and 3-(2-methoxyphenoxy)propylchloride (3.5 g, 17.4 mmoles) in acetonitrile (40 ml) was heated at 90°C for 4 hr. At the end of the reaction, the solvent was removed, and the solids were dissolved into dichloromethane (100 ml). The solution was washed with water and brine, then dried over MgSO₄. The crude material from the solution was combined with 1.2 g of crude material prepared in the same fashion (using 0.5 g of starting material). The combined material was purified by flash chromatography on a silica gel column (49 g, eluted with 0.5% diethylamine: 1% methanol:98.5% dichloromethane, 1^P). The fractions containing the pure product were pooled and concentrated down to a light oil (3.68 g). This oil was treated with fumaric acid (1.14 g, 9.8 mmoles) in ethanol (13 ml). The 6-fluoro-3-[1-[3-(2-methoxyphenoxy)propyl]-4-piperidinyl]-1,2-benzisoxazole fumarate crystals obtained weighed 4.01 g (60%), m.p. = 169-170°C.

| ANALYSIS: | | | |
|---|---|---|---|
| Calculated for C₂₂H₂₅FN₂O₃ . C₄H₄O₄: | 62.39%C | 5.84%H | 5.60%N |
| Found: | 62.37%C | 5.88%H | 5.60%N |

### EXAMPLE 19

### 1-[3-[4-(6-Fluoro-1,2-benzisoxazol-3-yl)-1-piperidinyl]propoxy]-4-methoxyphenyl]phenylmethanone

A stirred mixture of 6-fluoro-3-(4-piperidinyl)-1,2-benzisoxazole (2.01 g; 9.13 mmoles), K₂CO₃ (2.0 g), and 1-[3-(3-chloropropoxy)-4-methoxyphenyl]phenylmethanone (3.93 g; 11.3 mmoles) and acetonitrile (50 ml) was heated at reflux for 4 hr. At the end of the reaction, the solvent was evaporated and the residue was partitioned between water (150 ml) and dichloromethane (400 ml). The dichloromethane solution was washed with water and brine (100 ml), dried over MgSO₄, then concentrated to an oil. The purification was done by flash chromatography over a silica gel column (SiO₂, 40 g; eluted with dichloromethane, 300 ml; 1% methanol in dichloromethane, 850 ml). The material thus obtained as a colorless oil solidified on standing. Recrystallization from ethanol (150 ml) gave 1-[3-[4-(6-fluoro-1,2-benzisoxazol-3-yl)-1-piperidinyl]propoxy]-4-methoxyphenyl]phenylmethanone as white crystals, 3.07 g (63%), m.p. = 140-141°C.

| ANALYSIS: | | | |
|---|---|---|---|
| Calculated for C₂₉H₂₉FN₂O₄: | 71.30%C | 5.98%H | 5.73%N |
| Found: | 71.09%C | 5.98%H | 5.73%N |

### EXAMPLE 20

### 1-[4-[4-[4-(1H-indazol-3-yl)-1-piperidinyl]butoxy]-3-methoxyphenyl]ethanone

A mixture of 3-(4-piperidinyl)-1H-indazole (3.2 g, 0.016 mol), 1-[4-(4-bromobutoxy)-3-methoxyphenyl]ethanone (5.0 g, 0.016 mol), K₂CO₃ (2.2 g) and acetonitrile (100 ml) was stirred and refluxed for 6 hours. The reaction was poured into water and the resulting yellow solid that formed was collected to afford 5.3 g of product. The compound was recrystallized from acetonitrile and then from ethyl acetate to yield 3.0 g (45%) of a slightly yellow solid of 1-[4-[4-[4-(1H-indazol-3-yl)-1-piperidinyl]butoxy]-3-methoxyphenyl]ethanone, m.p. = 133-135°C.

| ANALYSIS: | | | |
|---|---|---|---|
| Calculated for C₂₅H₃₁N₃O₃: | 71.23%C | 7.41%H | 9.97%N |
| Found: | 70.85%C | 7.61%H | 9.81%N |

### EXAMPLE 21

### 1-[4-[2-[4-(6-Chloro-1,2-benzisoxazol-3-yl)-1-piperidinyl]ethoxy]-3-methoxyphenyl]ethanone

A stirred mixture of 6-chloro-3-(4-piperidinyl)-1,2 benzisoxazole (4.6 g, 0.019 mol); 1-(4-(2-chloroethoxy)-3-methoxyphenyl]ethanone (4.3 g, 0.019 mol), K₂CO₃ (2.8 g), a few crystals of KI and acetonitrile (120 ml) was refluxed for 16 hours. The reaction was filtered and the filtrate was concentrated to yield 8.0 g of yellow solid. The solid was chromatographed on a ®Waters Prep 500 LC (silica columns, eluting with methylene chloride/methanol, 5%). Concentration of the appropriate fractions yielded 3.2 g of a light yellow solid, which upon recrystallization from ethyl acetate afforded 2.3 g (28%) of 1-[4-[2-[4-(6-chloro-1,2-benzisoxazol-3-yl)-1-piperidinyl]ethoxy]-3-methoxyphenyl]ethanone as a pale yellow solid, m.p. = 133-135°C.

| ANALYSIS: | | | |
|---|---|---|---|
| Calculated for C₂₃H₂₅ClN₂O₄: | 64.41%C | 5.88%H | 6.53%N |
| Found: | 64.35%C | 5.87%H | 6.41%N |

### EXAMPLE 22

### 3-(3-Bromopropoxy-4-methoxyphenyl)phenylmethanone

A solution of 3-hydroxy-4-methoxybenzophenone (4.6 g, 20 mmoles) in dimethylformamide (35 ml) was treated with sodium hydride (600 mg, 25 mmoles) at 0°C for 20 minutes, then 1,3-dibromopropane (5 g, 24.7 mmoles) was added in one portion. The mixture was heated at 90°C for 1 hr, and then stirred at room temperature for 2 hr. At the end of the reaction, the mixture was poured into water (500 ml) and extracted with ethyl acetate (400 ml). The ethyl acetate solution was washed with water, brine and dried over anhydrous MgSO₄. The solvent was removed and the crude oil was purified by flash chromatography over a silica gel column (SiO₂, 85 g; eluted with 3:1 hexane:dichloromethane, 1.6 l; 3:7 hexane:dichloromethane, 1.4 l). The pure product thus obtained weighed 4.67 g, (66%) as an oil. Recrystallization twice from isopropyl ether (500 ml) gave analytically pure 3-(3-bromopropoxy-4-methoxyphenyl)phenylmethanone (2.42 g), m.p. = 81-83°C.

| ANALYSIS: | | |
|---|---|---|
| Calculated for C₁₇H₁₇BrO₃: | 58.47%C | 4.91%H |
| Found: | 58.63%C | 4.82%H |

### EXAMPLE 23

### 1-[3-[-3[4-(6-Fluoro-1,2-benzisoxazol-3-yl)-1-piperidinyl]propoxy]phenyl]ethanone fumarate

A mixture of 6-fluoro-3-(4-piperidinyl)-1,2-benzisoxazole hydrochloride (4.53 gm, 20.3 mmoles), K₂CO₃ (4.5 gm), 1-[3-(3-chloropropoxy)phenyl]ethanone (6.4 g, 29 mmoles) in acetonitrile (60 ml) was heated at reflux for 5 hr. At the end of the reaction, the solvent was removed and the residue was extracted into dichloromethane (300 ml). The inorganic insolubles were filtered off. The dichloromethane solution was concentrated to a small volume (10 ml) and purified on a flash chromatographic column (SiO₂, 75 g, eluted with dichloromethane, 900 ml; and 2% methanol in dichloromethane, 800 ml). The fractions containing the pure product were combined and concentrated to an oil (2.87 g, 35%). The oil was dissolved into ethanol and treated with a solution of fumaric acid (841 mg). Recrystallization (twice) from ethanol afforded 2.53 g of 1-[3-[3-[4-(6-fluoro-1,2-benzisoxazol-3-yl)-1-piperidinyl]propoxy]phenyl]ethanone fumarate as white crystals, m.p. = 172-174°C.

| ANALYSIS: | | | |
|---|---|---|---|
| Calculated for C₂₂H₂₅FN₂O₃.C₄H₄O₄ | 63.27%C | 5.70%H | 5.47%N |
| Found: | 63.04%C | 5.63%H | 5.43%N |

### Example 24

### 1-[4-[3-[4-(6-Fluoro-1,2-benzisoxazol-3-yl)-1-piperidinyl]propoxy]-2-methylphenyl]ethanone

A stirred mixture of 6-fluoro-3-(4-piperidinyl)-1,2 benzisoxazole hydrochloride (5.5 g, 21.6 mmoles), K₂CO₃(3.5 gm), 1-[4-(3-bromopropoxy)-2-methylphenyl]ethanone (4.83 g, 17.8 mmoles) in dimethylformamide (25 ml) and acetonitrile (75 ml) was heated at 120°C for 5 hr. At the end of the reaction, the solvent was removed and the residue was extracted into dichloromethane (300 ml) and the solution was washed with water and brine. The organic solution was dried and evaporated to a crude oil. The purification was done by flash chromatography over a silica gel column (80 g, eluted with dichloromethane, 1 l; 1% methanol: dichloromethane, 1.2 l; 2% methanol:dichloromethane, 1.2 l). The purest fractions were combined and afforded 2.91 g of solid. Recrystallization from dichloromethane and ethanol gave 1-[4-[3-[4-(6-fluoro-1,2-benzisoxazol-3-yl)-1-piperidinyl]propoxy]-2-methylphenyl]ethanone as off-white crystals: 2.42 g, m.p. = 113-114°C.

| ANALYSIS: | | | |
|---|---|---|---|
| Calculated for C₂₄H₂₇FN₂O₃: | 70.22%C | 6.63%H | 6.82%N |
| Found: | 70.13%C | 6.63%H | 6.77%N |

### EXAMPLE 25

### 1-[3-[3-[4-(6-Fluoro-1,2-benzisoxazol-3-yl)-1-piperidinyl]propoxy]-5-methylphenyl]ethanone

A mixture of 6-fluoro-3-(4-piperidinyl)-1,2-benzisoxazole hydrochloride (2.87 g, 11.23 mmoles), K₂CO₃ (2.5 g), 1-[2-(3-bromopropoxy)-5-methylphenyl]ethanone (3.74 g, 13.8 mmoles) in dimethylformamide (10 ml) and acetonitrile (50 ml) was heated at 95°C for 6 hr. At the end of the reaction, the solvent was concentrated and the mixture was extracted into dichloromethane (300 ml). The organic solution was washed with water and brine, dried over MgSO₄, then concentrated down to a crude oil. The purification was done by flash chromatography over a silica gel column (SiO₂, 60 g, eluted with 1% CH₃OH:dichloromethane: 1.2 l; 3% CH₃OH:dichloromethane: 600 ml). The material thus obtained was crystallized from a small volume of ether and hexane to provide 2.13 gm (46%) of off-white 1-[3-[3-[4-(6-fluoro-1,2-benzisoxazol-3-yl)-1-piperidinyl]propoxy]-5-methylphenyl]ethanone m.p. = 92-93°C.

| ANALYSIS: | | | |
|---|---|---|---|
| Calculated for C₂₄H₂₇FN₂O₃: | 70.22%C | 6.63%H | 6.82%N |
| Found: | 70.21%C | 6.69%H | 6.81%N |

### EXAMPLE 26

### N-[3-[3-[4-(6-Fluoro-1,2-benzisoxazol-3-yl)-1-piperidinyl]propoxy]-4-methoxyphenyl]acetamide hemifumarate

A mixture of 6-fluoro-3-(4-piperidinyl)-1,2-benzisoxazole hydrochloride (3.94 g, 15.4 mmoles), K₂CO₃ (3.67 g, 26.6 mmole), N-[3-(3-bromopropoxy)-4-methoxyphenyl]acetamide (5.56 g, 18.6 mmoles) in dimethylformamide (75 ml) and acetonitrile (100 ml) was heated at 100°C for 3 hr. At the end of the reaction, the solvent was concentrated and the mixture was extracted into dichloromethane (500 ml). The organic solution was washed with water (500 ml) and brine (400 ml), dried, then concentrated to a crude oil. The purification was effected by flash chromatography over a silica gel column (SiO₂, 65 g, eluted with 1% CH₃OH:dichloromethane, 1.2 l; and 3% CH₃OH:dichloromethane, 500 ml). The material thus obtained weighed 2.33 g (34.3%) as an oil. This material was dissolved in ethanol and treated with a solution of fumaric acid (661 mg) in ethanol. The N-[3-[3-[4-(6-fluoro-1,2 benzisoxazol-3-yl)-1-piperidinyl]propoxy]-4-methoxyphenyl]acetamide hemifumarate was obtained as off-white crystals weighing 2.17 g, m.p. = 205-206°C.

| ANALYSIS: | | | |
|---|---|---|---|
| Calculated for C₂₄H₂₈FN₃O₄·0.5 C₄H₄O₄: | 62.50%C | 6.05%H | 8.41%N |
| Found: | 62.30%C | 6.05%H | 8.32%N |

### EXAMPLE 27

### 6-Chloro-3-(1-piperazinyl)-1H-indazole

To a stirred suspension of 4-(6-chloro-1-phenylsulfonyl-1H-indazol-3-yl)-1-piperazinecarbonitrile (192.5 g, 0.479 mol) in dry tetrahydrofuran (3.5 l) under N₂ was added, dropwise, LiAlH₄ (958 ml of a 1.0 M solution of lithium aluminum hydride in tetrahydrofuran; 0.958 mol). After complete addition, the reaction was heated to reflux and stirred under N₂ for 4 hours. The reaction was cooled to 4° in an ice-salt bath and the excess lithium aluminum hydride was destroyed by the careful, dropwise addition of H₂O. The mixture was stirred vigorously for an additional 30 minutes and was then filtered through a coarse sintered glass funnel. The filter cake was washed well with tetrahydrofuran (3x500 ml) and then with methanol (2x500 ml) and the filtrate was concentrated to yield 151.0 g of a beige gum. Trituration with diethyl ether, afforded a solid, which was collected and dried to give 75.0 g (66%) of the desired indazole. A 4.0 g sample was recrystallized from toluene to yield 3.2 g, which was recrystallized again from toluene (utilizing decolorizing carbon) to provide 2.1 g (35%) of a beige, 6-chloro-3-(1-pipernzinyl)-1H-indazole solid, m.p. = 135-137°C.

| ANALYSIS: | | | |
|---|---|---|---|
| Calculated for C₁₁H₁₃ClN₄: | 55.82%C | 5.54%H | 23.67%N |
| Found: | 55.91%C | 5.54%H | 23.41%N |

### EXAMPLE 28

### 1-[4-[3-[4-(6-Fluoro-1H-indazol-3-yl)-1-piperidinyl]propoxy]-3-methoxyphenyl]ethanone

A stirred mixture of 6-fluoro-3-(4-piperidinyl)-1H-indazole (3.5 g, 0.016 mol), K₂CO₃ (2.2 g), 1-[4-(3-chloropropoxy)-3-methoxyphenyl]ethanone (3.8 g, 0.016 mol) and acetonitrile (90 ml) was refluxed for 16 hours. The reaction was poured into water and the resulting white solid, which precipitated from solution, was collected to afford 5.5 g of the desired product. The compound was recrystallized from dimethylformamide (twice) to afford 3.0 g (44%) of 1-[4-[3-[4-(6-fluoro-1H-indazol-3-yl)-1-piperidinyl]propoxy]-3-methoxyphenyl]ethanone as a white solid, m.p. = 202-204°C.

| ANALYSIS: | | | |
|---|---|---|---|
| Calculated for C₂₄H₂₈FN₃O₃: | 67.75%C | 6.63%H | 9.88%N |
| Found: | 67.59%C | 6.61%H | 9.96%N |

### EXAMPLE 29

### 1-[4-[3-[4-(6-Fluoro-1,2-benzisoxazol-3-yl)-1-piperidinyl]propoxy]-3-methylphenyl]ethanone hemifumarate

A stirred mixture of 6-fluoro-3-(4-piperidinyl)-1,2-benzisoxazole hydrochloride (3.0 g; 11.7 mmoles), K₂CO₃ (3.0 g), and 1-[4-(3-bromopropoxy)-3-methylphenyl]ethanone (3.19 g) in dimethylformamide (20 ml) and acetonitrile (50 ml) was heated at 95°C for 4 hr. At the end of the reaction, the solvent was concentrated down to about 30 ml, then partitioned between water (200 ml) and dichloromethane (300 ml). The dichloromethane solution was separated and washed with water and brine, then dried over MgSO₄. The crude product from the evaporated solution was purified by flash chromatography over a silica gel column (SiO₂, 60 g, eluted with 1% methanol in dichloromethane, 600 ml; 2% methanol in dichloromethane, 600 ml). The material thus obtained was a light yellow oil, weight: 2.07 g (43%). This oil was dissolved in ethanol and treated with a solution of fumaric acid (585 mg) in ethanol. The 1-[4-[3-[4-(6-fluoro-1,2-benzisoxazol-3-yl)-1-piperidinyl]propoxy]-3-methylphenyl]-ethanone hemifumarate crystals formed on cooling at 0°C. This was collected and weighed 1.5 g, m.p. = 185-187°C.

| ANALYSIS: | | | |
|---|---|---|---|
| Calculated for C₂₄H₂₇FN₂O₃.0.5 C₄H₄O₄: | 66.65%C | 6.24%H | 5.98%N |
| Found: | 66.69%C | 6.23%H | 5.95%N |

### EXAMPLE 30

### 1-[4-[3-[4-(6-Fluoro-1,2-benzisoxazol-3-yl)-1-piperidinyl]propoxy]phenyl]ethanone

A mixture of 6-fluoro-3(4piperidinyl)-1,2-benzisoxazole (3.27 g, 14.8 mmoles), K₂CO₃ (3 g), 1-[4-(3-bromopropoxy)phenyl]-ethanone (4.5 g, 17.5 mmoles) in acetonitrile (60 ml) was heated at reflux for 4 hr. The solvent was removed. The residue was dissolved in dichloromethane (300 ml) and washed with water and brine, then dried over MgSO₄. The crude product from the evaporated solution was purified by flash chromatography (SiO₂, 60 g; eluted with 1% methanol in dichloromethane, 1 liter). The purest fractions were combined and gave 2.8 g, 48%, of 1-[4-[3-[4-(6-fluoro 1,2-benzisoxazol-3-yl)-1-piperidinyl]propoxy)phenyl]ethanone, m.p. = 111-112°C.

| ANALYSIS: | | | |
|---|---|---|---|
| Calculated for C₂₃H₂₅FN₂O₃: | 69.68%C | 6.36%H | 7.07%N |
| Found: | 69.80%C | 6.38%H | 7.07%N |

### EXAMPLE 31

### 1-[4-[3-[4-(6-Chloro-1H-indazol-3-yl)-1-piperazinyl]propoxy]-3-methoxyphenyl]ethanone

A mixture of 6-chloro-[3-(1-piperazinyl)]-1H-indazole (3.4 g, 0.014 mol), K₂CO₃ (2.5 g, 0.018 mol), 1-[4-(3-chloropropoxy)-3-methoxyphenyl]ethanone (3.8 g, 0.016 mol), KI (200 mg), and acetonitrile (125 ml) was stirred at reflux under N₂ for 30 hours. After standing at room temperature for 40 hours, the reaction was filtered and the filter cake was washed well with acetonitrile. The filtrate was concentrated to an oily solid, which was partitioned between water and ethyl acetate. The ethyl acetate extract was washed with water, dried with MgSO₄, and concentrated to yield 6.9 g of a dark oil, which solidified after 2 days under vacuum. The product was purified by preparative HPLC (®Waters Associates Prep LC/system 500 utilizing 2 silica gel columns and 6% methanol/methylene chloride as eluent) to yield 4.2 g. The material was recrystallized from ethanol to yield 3.4 g of glistening, beige, 1-[4-[3-[4-(6-chloro-1H-indazol-3-yl)-1-piperazinyl]propoxy]-3-methoxyphenyl]ethanone crystals, m.p. = 132-134°C.

| ANALYSIS: | | | |
|---|---|---|---|
| Calculated for C₂₃H₂₇ClN₄O₃: | 62.37%C | 6.14%H | 12.65%N |
| Found: | 62.49%C | 6.16%H | 12.60%N |

### EXAMPLE 32

### 1-[4-[4-[4-(1,2-Benzisothiazol-3-yl)-1-piperazinyl]butoxy]-3-methoxyphenyl]ethanone

A mixture of 3-(1-piperazinyl)-1,2-benzisothiazole (4.0 g, 0.0182 mol), 1-[4-(4-bromobutoxy)-3-methoxyphenyl]ethanone (6.0 g, 0.0200 mol), K₂CO₃ (3.0 g, 0.0218 mol), KI (200 mg), and acetonitrile (125 ml) was stirred at reflux under N₂ for 5 hours. Most of the solvent was removed in vacuo and the resultant gummy residue was partitioned between ethyl acetate and water. The organic extract was washed with water, dried with MgSO₄, and concentrated to yield 7.8 g. Purification by preparative HPLC (Waters Associates Prep LC/System 500, utilizing 2 silica gel columns and 4% methanol-methylene chloride as eluent) afforded 6.5 g of a damp, off-white solid. The product was recrystallized twice from toluene to provide 3.1 g (39%) of 1-[4-[4-[4-(1,2-benzisothiazol-3-yl)-1-piperazinyl]butoxy]-3-methoxyphenyl]ethanone as a white solid, m.p. = 114-116°C.

| ANALYSIS: | | | |
|---|---|---|---|
| Calculated for C₂₄H₂₉N₃O₃S: | 65.58%C | 6.65%H | 9.56%N |
| Found: | 65.74%C | 6.66%H | 9.54%N |

### EXAMPLE 33

### 4-[3-[4-(6-Fluoro-1,2-benzisoxazol-3-yl-)-1-piperidinyl]propoxy]-3-methoxybenzonitrile

A mixture of 6-fluoro-3-(4-piperidinyl)-1,2-benzisoxazole (3.0 g, 13.6 mmoles), K₂CO₃ (2.8 g), 4-(3-bromopropoxy)3-methoxybenzonitrile (4.0 gm, 14.8 mmoles) in acetonitrile (70 ml) was heated at reflux for 3 hr. At the end of the reaction, the solvent was removed on a rotary evaporator. The organic material was extracted into dichloromethane (250 ml) and the inorganics were filtered off. The dichloromethane solution was concentrated to a crude oil. The purification was done by flash chromatography over a silica gel column (SiO₂, 55 gm; eluted with dichloromethane, 600 ml; 1% methanol in dichloromethane, 600 ml). The material thus obtained was crystallized from a small amount of dichloromethane. Recrystallization from ethanol (25 ml) provided 3.8 gm (68%) of 4-[3-[4-(6-fluoro-1,2-benzisoxazol-3-yl)-1-piperidinyl]propoxy]-3-methoxybenzonitrile as white crystals, m.p. = 107-108°C.

| ANALYSIS: | | | |
|---|---|---|---|
| Calculated for C₂₃H₂₄FN₃O₃: | 67.47%C | 5.91%H | 10.26%N |
| Found: | 67.32%C | 5.90%H | 10.24%N |

### EXAMPLE 34

### 1-[4-[4-[4-(6-Fluoro-1H-indazol-3-yl)-1-piperidinyl]butoxy]-3-methoxyphenyl]ethanone

A stirred mixture of 6-fluoro-3-(4-piperidinyl)-1H-indazole (1.9 g, 0.0086 mol), 1-[4-(4-bromobutoxy)-3-methoxyphenyl]ethanone (2.6 g, 0.0086 mol), K₂CO₃ (1.2 g), and acetonitrile (75 ml) was refluxed for 6 hr. The reaction was poured into water and a white solid settled from solution. This was collected, dried and afforded 3.2 g of product. The product was recrystallized from ethanol to yield 2.7 g (71%) of 1-[4-[4-[4-(6-fluoro-1H-indazol-3-yl)-1-piperidinyl]butoxy]-3-methoxyphenyl]ethanone as glistening white flakes, m.p. = 158-160°C.

| ANALYSIS: | | | |
|---|---|---|---|
| Calculated for C₂₅H₃₀FN₃O₃: | 68.32%C | 6.88%H | 9.56%N |
| Found: | 68.00%C | 6.93%H | 9.51%N |

### EXAMPLE 35

### 1-[4-[3-[4-(1-Benzoyl-6-fluoro-1H-indazol-3-yl)-1-piperazinyl]propoxy]-3-methoxyphenyl]ethanone sesquifumarate

A mixture of 1-[4-[3-[4-(6-fluoro-lH-indazol-3-yl)-1-piperazinyl]propoxy]-3-methoxyphenyl]ethanone (3.2 g, 0.0075 mol) and benzoyl chloride (15 ml) was heated on a steam bath for 15 min. The reaction was allowed to cool and ether was added. The insoluble off-white compound was harvested to yield 4.4 g of the product as a hydrochloride salt. The salt was converted to free base with aqueous ammonium hydroxide, and after extractive workup with methylene chloride, 3.0 g of the free base was isolated as a white solid. The free base was dissolved in ethyl acetate and fumaric acid (0.72 g, 1.1 eq) was added and the mixture heated on the steam bath for 15 min. After standing at ambient temperature for 4 days, 2.0 g of an off-white fumarate salt was collected, while concentration of the filtrate afforded an additional 1.0 g of the salt. Recrystallization, first from ethyl acetate, and then from ethanol yielded 1.4 g (26%) of 1-[4-[3-[4-(1-benzoyl-6-fluoro-1H-indazol-3-yl)-1-piperazinyl]propoxy]-3-methoxyphenyl]ethanone sesquifumarate, m.p. = 138-140°C.

| ANALYSIS: | | | |
|---|---|---|---|
| Calculated for C₃₀H₃₁FN₄O₄·1.5C₄H₄O₄: | 61.35%C | 5.29%H | 7.95%N |
| Found: | 61.68%C | 5.31%H | 8.25%N |

### EXAMPLE 36

### 1-[4-[4-[4-(6-Chlorol-1H-indazol-3yl)-1-piperazinyl]butoxy]-3-methoxyphenyl]ethanone

A mixture of 6-chloro-[3-(1-piperazinyl)]-1H-indazole (4.0 g, 0.017 mol), K₂CO₃ (2.8 g, 0.020 mol), 1-[4-(4-bromobutoxy)-3-methoxyphenyl]ethanone (5.7 g, 0.019 mol), KI (100 mg) and acetonitrile (125 ml) was stirred at reflux under nitrogen for 18 hrs. The cooled reaction was poured into water and the resultant off-white solid was collected by filtration and dried to yield 7.0 g. The compound was recrystallized twice from toluene to yield 6.2 g. Further purification by preparative HPLC (®Waters Associates Prep LC/System 500, utilizing 5% methanol/methylene chloride as eluent and 2 silica gel columns) afforded 5.3 g of glistening, beige crystals, which were recrystallized four times from toluene to yield 3.1 g of a white solid. Analytically pure material was obtained by a subsequent recrystallization from dimethylformamide to afford 2.5 g (32%) of 1-[4-[4-[4-(6-chloro-1H-indazol-3-yl)-1-piperazinyl]butoxy]-3-methoxyphenyl]ethanone as an off-white powder, m.p. = 189-191°C.

| ANALYSIS: | | | |
|---|---|---|---|
| Calculated for C₂₄H₂₉ClN₄O₃: | 63.08%C | 6.40%H | 12.26%N |
| Found: | 62.86%C | 6.57%H | 12.49%N |

### EXAMPLE 37

### 1-[4-[3-[4-(1,2-Benzisothiazol-3-yl)-1-piperazinyl] propoxy]-3-methoxyphenyl]ethanone hemifumarate

A mixture of 3-(1-piperazinyl)-1,2-benzisothiazole (4.0 g, 0.0182 mol), K₂CO₃ (3.0 g, 0.0218 mol), KI (200 mg), 1-[4-(3-chloropropoxy)-3-methoxyphenyl]ethanone (5.3 g, 0.0200 mol), and acetonitrile (125 ml) was stirred at reflux under N₂ for 26 hours. The cooled reaction was filtered and the filter cake was washed well with acetonitrile. The filtrate was concentrated to afford 10.7 g of an oily residue, which was extracted with ethyl acetate. The ethyl acetate extract was washed with water, dried with MgSO₄ and concentrated to yield 8.0 g of a dark oil. The oil was purified by preparative HPLC (®Waters Associates Prep LC/System 500, utilizing 2 silica gel columns and 3% methanol/methylene chloride as eluent). Concentration of appropriate fractions provided 4.6 g of a red oil, which solidified upon standing. A 3.4 g sample was taken up in ethyl acetate (100 ml) and fumaric acid (0.95 g) was added. The mixture was stirred at a mild reflux for 1 hour and then at ambient for 1.5 hrs. The resultant beige solid was collected by filtration and dried to yield 4.0 g. The product was recrystallized twice from ethanol to provide 2.7 g (27%) of 1-[4-[3-[4-(1,2-benzisothiazol-3-yl)-1-piperazinyl)propoxy]-3-methoxyphenyl]ethanone hemifumarate as a beige powder, m.p. = 186-188°C.

| ANALYSIS: | | | |
|---|---|---|---|
| Calculated for C₂₃H₂₇N₃O₃S·0.5 C₄H₄O₄: | 62.09%C | 6.06%H | 8.69%N |
| Found: | 62.01%C | 6.06%H | 8.68%N |

### EXAMPLE 38

### 1-[3,5-Dibromo-4-[3-[4-(6-fluoro-1,2-benzisoxazol-3-yl)-1-piperidinyl]propoxy]phenyl]ethanone

A stirred mixture of 6-fluoro-3-(4-piperidinyl)-1,2-benzisoxazole (2.0 g, 9.0 mmoles), K₂CO₃ (1.3 g), and 1-[4-(3-bromopropoxy)-3,5-dibromophenyl]ethanone (2.65 g, 9.0 mmoles) and acetonitrile (50 ml) was heated at reflux for 3 hr. At the end of the reaction, the solvent was evaporated and the residue was extracted into dichloromethane (150 ml). The insolubles were filtered off. The dichloromethane solution was concentrated down to an oil. The purification was done by flash chromatography on a silica gel column (SiO₂, 47 g; eluted with dichloromethane, 300 ml; 1% methanol in dichloromethane, 600 ml). The material thus purified as a colorless oil, solidified on standing. Recrystallization from ethanol gave 1-[3,5-dibromo-4-[3-[4-(6-fluoro-1,2-benzisoxazol-3-yl)-1-piperidinyl]propoxy]phenyl]ethanone as white crystals (2.93 g, 57%), m.p. = 102-103°C.

| ANALYSIS: | | | |
|---|---|---|---|
| Calculated for C₂₃H₂₃Br₂FN₂O₃: | 49.84%C | 4.18%H | 5.05%N |
| Found: | 49.91%C | 4.11%H | 4.98%N |

### EXAMPLE 39

### 1-[4-[2-[4-(1,2-Benzisothiazol-3-yl)-1-piperazinyl]ethoxy]-3-methoxyphenyl]ethanone

A mixture of 3-(1-piperazinyl)-1,2-benzisothiazole (4.0g, 0.0182 mol), 1-[4-(2-chloroethoxy)-3-methoxyphenyl]ethanone (4.3g, 0.0200 mol), K₂CO₃ (3.0g, 0.0218 mol), acetonitrile (125 ml) and a catalytic amount of KI was heated to reflux and stirred under nitrogen for 24 hours. At this point, an additional amount of K₂CO₃ (1.0 g, 0.0072 mol) and alkylating agent (0.4g, 0.0017 mol) was added to the reaction mixture and heating at reflux was resumed for 24 hours. The reaction was cooled to ambient temperature and filtered. The filter cake was washed with acetonitrile and the filtrate was concentrated to afford a dark oil. The oil was extracted with methylene chloride, and the organic extract was washed with water, dried with MgSO₄ and concentrated to yield 9.2g of an oil. Purification by preparative HPLC (®Waters Associates Prep LC/System 500 utilizing 2 silica gel columns and 3% methanol/methylene chloride as eluent) provided 3.8g of a soft, beige gum, which readily solidified. The compound was recrystallized twice from ethanol to give 2.1g (28%) of 1-[4-[2-[4-(1,2-benzisothiazol-3-yl)-1-piperazinyl]ethoxy]-3-methoxyphenyl]ethanone as a beige solid, m.p. = 98-100°C.

| ANALYSIS: | | | |
|---|---|---|---|
| Calculated for C₂₂H₂₅N₃O₃S: | 64.21%C | 6.12%H | 10.21%N |
| Found: | 64.05%C | 6.09%H | 10.12%N |

### EXAMPLE 40

### 6-Fluoro-3-[1-(3-phenoxypropyl)-4-piperidinyl]-1,2-benzisoxazole

A mixture of 6-fluoro-3-(4-piperidinyl)-1,2-benzisoxazole (4.0 g, 0.0182 mol), K₂CO₃ (3.0 g, 0.0218 mol), KI (100 mg), 3-chloropropoxybenzene (3.4 g, 0.0200 mol), and acetonitrile was stirred at reflux under nitrogen for 30 hours. The reaction was poured into water and the aqueous mixture was extracted with ethyl acetate. The ethyl acetate extract was washed with brine, dried with MgSO₄ and concentrated to afford 6.2g of a damp, beige solid. The compound was recrystallized twice from ethanol to yield (47%) of 6-fluoro-3-[1-(3-phenoxypropyl)-4-piperidinyl]-1,2-benzisoxazole as a light beige solid, m.p. = 78-80°C.

| ANALYSIS: | | | |
|---|---|---|---|
| Calculated for C₂₁H₂₃FN₂O₂: | 71.17%C | 6.54%H | 7.90%N |
| Found: | 71.00%C | 6.52%H | 7.81%N |

### EXAMPLE 41

### 1-[4-[2-[4-(6-Chloro-1H-indazol-3-yl)-piperazinyl] ethoxy]-3-methoxyphenyl]ethanone

A mixture of 6-chloro-[3-(1-piperazinyl)]-1H-indazole (2.1g, 0.0089 mol), K₂CO₃ (1.5g, 0.0107 mol), KI (100mg), 1-[4-(2-chloroethoxy)-3-methoxyphenyl]ethanone (2.2g, 0.0098 mol) and acetonitrile (70 ml) was stirred at reflux for 48 hours under N₂. The cooled reaction was poured into water and the aqueous mixture was extracted with ethyl acetate. The organic extract was washed with water, dried with MgSO₄ and concentrated to yield 6.0g of a light yellow oil. The oil was purified by preparative HPLC (®Waters Associates prep LC/System 500, employing 2 silica gel columns and 5.5% methanol/methylene chloride as eluent). Concentration of later fractions provided 1.6g of an off-white solid. This was combined with an additional sample (3.4g total) and two consecutive recrystallizations from ethanol yielded 2.1g (23%) of 1-[4-[2-[4-(6-chloro-1H-indazol-3-yl)-1-piperazinyl]ethoxy]-3-methoxyphenyl]ethanone as an off-white solid, m.p. = 154-156°C.

| ANALYSIS: | | | |
|---|---|---|---|
| Calculated for C₂₂H₂₅ClN₄O₃: | 61.61%C | 5.88%H | 13.06%N |
| Found: | 61.66%C | 5.87%H | 13.06%N |

### EXAMPLE 42

### 1-[4-[3-[4-(6-Fluoro-1,2-benzisoxazol-3-yl)-1-piperidinyl]propoxy]-3-methoxyphenyl]-2,2,2-trifluoroethanone

A mixture of 6-fluoro-3-(4-piperidinyl)-1,2-benzisoxazole (1.5 g, 0.0067 mol), 1-[4-(3-chloropropoxy)-3-methoxyphenyl]-2,2,2-trifluorethanone (2.0 g, 0.0067 mol), K₂CO₃ (0.88 g), KI (0.1 g) and acetonitrile (50 ml) was stirred and refluxed for 16 h. After cooling, the reaction was poured into water and the aqueous mixture extracted with ethyl acetate. The extract was washed (H₂O), dried (MgSO₄), and the solvent was concentrated to an oil, which upon evacuation at high vacuum afforded 3.2 g of a waxy solid. The solid was chromatographed on a ®Waters preparative LC (silica columns, eluting with 3% methanol-dichloromethane). Concentration of the appropriate fractions gave 1.8 g (56%) of 1-[4-[3-[4-(6-fluoro-1,2-benzisoxazol-3-yl)-1-piperidinyl]propoxy]-3-methoxyphenyl]-2,2,2-trifluoromethylethanone solid, m.p. = 94-96°C.

| ANALYSIS: | | | |
|---|---|---|---|
| Calculated for C₂₄H₂₄F₄N₂O₄: | 60.00%C | 5.03%H | 5.83%N |
| Found: | 60.01%C | 5.06%H | 5.68%N |

### EXAMPLE 43

### 1-[4-[3-[4-(6-Fluoro-1,2-benzoisoxazol-3-yl]-1-piperidinyl]propoxy]-3-methylmercaptophenyl]ethanone

A stirred mixture of 6-fluoro-3-(4-piperidinyl)-1,2-benzisoxazole (1.88 g, 8.5 mmoles), K₂CO₃ (1.8 g) and 1-[4-(3-bromopropoxy)-3-methylmercaptophenyl]ethanone (2.3 g, 7.6 mmole) in acetonitrile (100 ml) was heated at reflux for 4 hr. At the end of the reaction, the solvent was concentrated, then diluted with dichloromethane (250 ml). The insolubles were filtered off. The dichloromethane solution was concentrated to dryness as an oil. Purification was effected by flash chromatography on a silica gel column (SiO₂, 54 g, eluted with dichloromethane, 500 ml; 1% methanol:dichloromethane, 1.1 l). The purest fractions were combined to give a colorless oil which solidified to an off-white solid (2.4 g). Recrystallization from ethanol (100 ml) yielded 1-[4-[3-[4-(6-fluoro-1,2-benzoisoxazol-3-yl]-1-piperidinyl]propoxy]-3-methylmercaptophenyl]ethanone as off-white needle crystals, 2.15 gm, m.p. = 150-152°C.

| ANALYSIS: | | | |
|---|---|---|---|
| Calculated for C₂₄H₂₇FN₂O₃S: | 65.14%C | 6.15%H | 6.33%N |
| Found: | 65.09%C | 6.10%H | 6.25%N |

### EXAMPLE 44

### 1-[4-(3-Bromopropoxy-3,5-bromophenyl]ethanone

A stirred mixture of 3-bromo-4-hydroxyacetophenone (4.5 g, 21.2 mmoles), K₂CO₃ (4 g) and 1,3-dibromopropane (7.6 g) in acetonitrile (200 ml) was heated at reflux for 2 hr. At the end of the reaction, the solvent was removed and the residue was dissolved in dichloromethane (400 ml) and filtered. The dichloromethane solution was concentrated to an oil. The oil was added to isopropyl ether and stirred to cause crystallization (4.1 g; 58%). The solid was recrystallized from isopropyl ether to give 3.5 g of 1-[4-(3-bromopropoxy)-3-bromophenyl]ethanone as glistening crystals, m.p. = 83-84°C.

| ANALYSIS: | | |
|---|---|---|
| Calculated for C₁₁H₁₂Br₂O₂: | 39.31%C | 3.60%H |
| Found: | 39.80%C | 3.55%H |

### EXAMPLE 45

### 1-[4-(3-Bromopropoxy)-3,5-dibromophenyl]ethanone

A stirred mixture of 3,5-dibromo-4-hydroxyacetophenone (3.0 g, 10.1 mmole), K₂CO₃ (2.8 g, 20.3 mmoles), 1,3-dibromopropane (4.0 g, 19.8 mmoles) in acetonitrile (100 ml) was heated at reflux for 5 hr. The solvent was removed. The crude product was extracted into dichloromethane (150 ml) and the insoluble inorganics were filtered off. The solution was concentrated to dryness again. Purification was carried out by flash chromatography on silica gel (45 g, SiO₂; eluted with 1:1 hexane:dichloromethane). The material thus obtained (2.8 g) was recrystallized twice from isopropyl ether to give analytically pure 1-[4-(3-bromopropoxy)-3,5-dibromophenyl]ethanone, m.p. = 87-88°C.

| ANALYSIS | | |
|---|---|---|
| Calculated for C₁₁H₁₁Br₃O₂: | 31.84%C | 2.67%H |
| Found: | 31.97%C | 2.63%H |

### EXAMPLE 46

### 1-[4-[4-[4-(1,2-Benzisothiazol-3-yl)-1-piperidinyl] butoxy]-3-methoxyphenyl]ethanone

A stirred mixture of 3-(4-piperidinyl)-1,2-benzisothiazole (2.6 g, 0.0119 mol), 1-[4-(4-bromobutoxy)-3-methoxyphenyl]-ethanone (3.9 g, 0.0131 mol), K₂CO₃ (2.0 g, 0.0143 mol), KI (200 mg) and acetonitrile (125 ml) was stirred at reflux under nitrogen for 18 hours. The reaction was cooled to ambient temperature and filtered. The filter cake was washed well with fresh acetonitrile and the filtrate was concentrated to yield a wet, brown solid. The residue was diluted with water and the aqueous suspension was extracted with methylene chloride. The organic extract was washed with water, dried with MgSO₄ and concentrated to afford 6.5 g of a dark oil. The oil was purified by preparative HPLC (®Waters Associates prep LC/System 500, utilizing 2 silica gel columns and 5% methanol/methylene chloride) to give 4.5 g of a beige solid. A 3.1 g (0.0071 mol) sample was taken up in absolute ethanol (80 ml) and oxalic acid (0.67 g, 0.0074 mol) was added. The solution was refluxed mildly on a steam bath for 45 minutes and was then stirred at ambient temperature for 1 hour. The resultant suspension was diluted with anhydrous ether (150 ml) and stirred for 5 minutes. The solid was collected and dried to afford 3.1 g of a light, beige solid. The salt was recrystallized from ethanol to yield 2.8 g. The compound was converted back to the free base with 50% NaOH to give 2.4 g, which was immediately recrystallized from ethanol to provide 1.5 g (29%) of 1-[4-[4-[4-(1,2-benzisothiazol-3-yl)-1-piperidinyl]butoxy]-3-methoxyphenyl]ethanone as a beige powder, m.p. = 78-80°C.

| ANALYSIS: | | | |
|---|---|---|---|
| Calculated for C₂₅H₃₀N₂O₃S: | 68.46%C | 6.91%H | 6.39%N |
| Found: | 68.34%C | 6.85%H | 6.33%N |

### EXAMPLE 47

### [4-[3-[4-(6-Fluoro-1,2-benzisoxazol-3-yl)-1-piperidinyl]propoxy]-3-methoxyphenyl]phenylmethanone

A mixture of 6-fluoro-3-(4-piperidinyl)-1,2-benzisoxazole (2.2 g, 10 mmoles), K₂CO₃ (2.3 g) and 1-[4-(3-bromopropoxy)-3-methoxyphenyl]phenylmethanone (3.47 g, 10 mmoles) in acetonitrile (100 ml) was heated at reflux for 3 hours. At the end of reaction, the acetonitrile was concentrated and the mixture was extracted into dichloromethane (200 ml). The insolubles were filtered off and the solvent was evaporated to an oil. Purification was carried out by flash chromatography over a silica gel column (SiO₂ 50 g; eluted with dichloromethane, 600 ml; 1% methanol:dichloromethane, 600 ml; 2% methanol: 98% dichloromethane, 600 ml). The fractions containing the pure product were combined and concentrated to give 4.24 g (87%) of an off-white solid. Recrystallization from ethanol (75 ml) gave 3.9 g of [4-[3-[4-(6-fluoro-1,2-benziosoxazol-3-yl)-1-piperidinyl]propoxy]-3-methoxyphenyl]phenylmethanone as off-white crystals, m.p. = 128-130°C.

| ANALYSIS: | | | |
|---|---|---|---|
| Calculated for C₂₉H₂₉FN₂O₄: | 71.30%C | 5.98%H | 5.73%N |
| Found: | 71.31%C | 5.99%H | 5.75%N |

### EXAMPLE 48

### 1-[3-Bromo-4-[3-[4-(6-fluoro-1,2-benziosoxazol-3-yl)-1-piperidinyl]propoxyl]phenyl]ethanone

A mixture of 6-fluoro-3-(4-piperidinyl)-1,2-benzisoxazole (2.1 g, 9.5 mmole), K₂CO₃ (2.0 g) 1-[3-bromo-4-(3-bromopropoxy)phenyl]ethanone (3.1 g, 9.2 mmoles) in acetonitrile (100 ml) was heated at reflux for 3 hours. At the end of reaction, the solvent was concentrated and the mixture was extracted into dichloromethane (200 ml). The insolubles were filtered off. The dichloromethane was concentrated again. The crude residue was purified by flash chromatography over a silica gel column (SiO₂, 49 g; eluted with dichloromethane, 500 ml; 1% methanol:dichloromethane, 600 ml; 3% methanol: 97% dichloromethane, 600 ml). The material thus obtained (3.26 g, 72%) was recrystallized from ethanol (40 ml) to give 1-[3-bromo-4-[3-[4-(6-fluoro-1,2-benziosoxazol-3-yl)-1-piperidinyl]propoxy]pheny]ethanone as light yellow crystals (3.0 g), m.p. = 126-128°C.

| ANALYSIS: | | | |
|---|---|---|---|
| Calculated for C₂₃H₂₄BrFN₂O₃: | 58.12%C | 5.09%H | 5.89%N |
| Found: | 57.64%C | 5.35%H | 5.55%N |

### EXAMPLE 49

### 3-[1-[3-[4-(1-Ethoxyethyl)-2-methoxyphenoxy]propyl]-4-piperidinyl]-6-fluoro-1,2-benzisoxazole hydrochloride

To a mixture of 4-[3-[4-(6-fluoro-1,2-benzisoxazol-3-yl)-1-piperidinyl]propoxy]-3-methoxy-^a-methylbenzenemethanol (3.8g, 0.089 mol) in pyridine (25 ml) was added acetic anhydride (5 ml). Tne mixture was warmed briefly on the steam bath to effect solution, and then the reaction was allowed to stand at ambient temperature for 16 hours. Most of the pyridine was evaporated under reduced pressure and the resultant oil was diluted with water. The aqueous solution was made basic with dilute NaOH, and subsequently extracted with ethyl acetate. The organic extract was washed (water), dried (MgSO₄), and the solvent concentrated to give 3.7 g of the O-acetyl derivative as a colorless oil. The compound was dissolved in diethyl ether and ethereal HCl was added to precipitate a gum-like hydrochloride salt, which upon treatment with refluxing ethyl acetate afforded 3.4 g of a crystalline salt, m.p. 143-145°C. Attempting to recrystallize the salt from ethanol:diethyl ether resulted in displacement of the acetate to afford the ethyl ether. The salt of this product (2.8 g) was recrystallized from ethanol:diethyl ether to yield 2.1 g (48%) of 3-[1-[3-[4-(1-ethoxyethyl)-2-methoxyphenoxy]propyl]-4-piperidinyl]-6-fluoro-1,2-benzisoxazole hydrochloride, m.p. = 139-141°C.

| ANALYSIS | | | |
|---|---|---|---|
| Calculated for C₂₆H₃₃FN₂O₄.HCl: | 63.34%C | 6.95%H | 5.68%N |
| Found: | 63.06%C | 6.80%H | 5.63%N |

### EXAMPLE 50

### 3-[1-[3-[4-(1-Acetoxyethyl)-2-methoxyphenoxy]propyl]-4-piperidinyl]-6-fluoro-1,2-benzisoxazole fumarate

A mixture of 4-[3-[4-(6-fluoro-1,2-benzisoxazol-3-yl)-1-piperidinyl]-3-methoxy-^a-methylbenzenemethanol (4.8 g, 0.011 mol) in pyridine (45 ml) was warmed briefly to effect solution and then acetic anhydride (6.3 ml) was added. The reaction stood at ambient temperature for 16 hours, was concentrated in vacuo, and the colorless oil that remained was dissolved in water. The aqueous solution was made basic with saturated K₂CO₃ solution, and the mixture was extracted with diethyl ether. The extract was washed (water), dried (MgSO₄) and concentrated to afford 5.2 g of a thick, colorless oil. The oil (4.8 g) was dissolved in anhydrous diethyl ether and fumaric acid (1.2 g, 0.01 mol) was added. The mixture was stirred at ambient temperature for 4 hours, and then was permitted to stand at ambient temperature for 16 hours. The resultant white, 3-[1-[3-[4-(1-acetoxyethyl)-2-methoxyphenoxy]propyl-4-piperidinyl]-6-fluoro-1,2-benzisoxazole fumarate was collected and afforded 3.0 g of material. The filtrate was treated with an additional amount of fumaric acid (0.3 g) and 0.9 g more of 3-[1-[3-[4-(1-acetoxyethyl)-2-methoxyphenoxy]propyl]-4-piperidinyl-6-fluoro-1,2-benzisoxazole fumarate was harvested. The two batches were combined and recrystallized from acetonitrile (twice) to yield 2.3 g (43%) of the acetate, m.p.=150-152°C.

| ANALYSIS: | | | |
|---|---|---|---|
| Calculated for C₂₆H₃₁FN₂O₃·C₄H₄O₄: | 61.43%C | 6.01%H | 4.78%N |
| Found: | 61.06%C | 5.87%H | 4.73%N |

### EXAMPLE 51

### 1-[4-[3-[4-(6-fluoro-1,2-benzisoxazol-3-yl)-1-piperidinyl]propoxy]-3-methoxyphenyl]pentanone

A mixture of 6-fluoro-3-(4-piperidinyl)-1,2-benzisoxazole (2.2 g, 0.01 mole), K₂CO₃ (3 g), 1-[4-(3-bromopropoxy)-3-methoxyphenyl]pentanone (3.7 g, 0.0113 mole) in acetonitrile (140 ml) was heated at reflux for 4 hours. At the end of the reaction, the mixture was cooled and filtered. The filtrate was concentrated to an oil. Purification was performed by flash chromatography over a silica gel column (SiO₂, 55 g; eluted with 1% methanol in dichloromethane, 600 ml; 3% methanol: 97% dichloromethane, 400 ml). The fractions containing pure product were pooled and concentrated to a solid (4.3 g, 91%). Recrystallization from ethanol (10 ml) gave a powdery solid of 1-[4-[3-[4-(6-fluoro-1,2-benzisoxazol-3-yl)-1-piperidinyl-3-propoxy]-3-methoxyphenyl]pentanone (3.22 g), m.p. = 79-80°C.

| ANALYSIS: | | | |
|---|---|---|---|
| Calculated for C₂₇H₃₃FN₂O₄: | 69.21%C | 7.10%H | 5.98%N |
| Found: | 69.00%C | 6.94%H | 6.39%N |

### EXAMPLE 52

### 2-[3-[4-(6-Fluoro-1,2-benzisoxazol-3-yl)-1-piperidinyl]propoxy]-N-methylbenzenamine hemifumarate

A mixture of 6-fluoro-3-(4-piperdinyl)-1,2-benzisoxazole (2.5 g, 0.0114 mol), K₂CO₃ (1.8 g, 0.0130 mol), 4-(3-chloropropoxy)-2-methylaminobenzene (2.4 g, 0.0120 mol) and acetonitrile (100 ml) was stirred at reflux for 18 hours. The reaction was cooled to ambient temperature and was poured into water. The aqueous mixture was extracted with ethyl acetate and the ethyl acetate extract was washed with water, dried with MgSO₄, and concentrated to yield 4.1 g of a brown oil. The oil was purified by preparative HPLC (®Waters Associates prep LC/System 500, utilizing 2 silica gel columns and eluting with 4% methanol-methylene chloride). Concentration of appropriate fractions yielded 2.45 g of a beige oil. The product was taken up in ethyl acetate (50 ml) and fumaric acid (0.78 g) was added. The mixture was stirred at mild reflux for 45 minutes and then at ambient temperature for 1.5 hours. The product was isolated by vacuum filtration to provide 2.5 g of a pale yellow solid. Recrystallization from ethanol afforded 2.0 g (40%) of 2-[3-[4-(6-fluoro-1,2-benzisoxazol-3-yl)-1-piperidinyl]propoxy]-N-methylbenzenamine hemifumarate as beige crystals, m.p. = 180-182°C.

| ANALYSIS: | | | |
|---|---|---|---|
| Calculated for C₂₂H₂₆FN₃O₂·0.5C₄H₄O₄: | 65.28%C | 6.40%H | 9.52%N |
| Found: | 65.08%C | 6.35%H | 9.45%N |

### EXAMPLE 53

### 3-[1-[3-(4-Bromo-2-methoxyphenoxy)propyl]-4-piperidinyl]-6-fluoro-1,2-benzisoxazole

A mixture of 6-fluoro-3-(4-piperidinyl)-1,2-benzisoxazole (2.6 g, 0.0117 mol), K₂CO₃ (2.0 g, 0.0144 mol), 4-(3-chloropropoxy)-3-methoxybromobenzene (3.6 g, 0.0129 mol), acetonitrile (100 ml), and a few KI crystals was stirred at reflux under nitrogen for 18 hours. The cooled reaction was poured into water and the aqueous mixture was extracted with ethyl acetate. The ethyl acetate extract was washed with water, dried with MgSO₄, and concentrated to yield 5.0 g of a green oil. The sample was purified by preparative HPLC (®Waters Associates prep LC/System 500, utilizing 2 silica gel columns and 4% methanol-methylene chloride as eluent). Concentration of appropriate fractions afforded 3.15 g of a tan solid. The compound was recrystallized twice from ethanol to yield 2.0 g (37%) of 3-[1-[3-(4-bromo-2-methoxyphenoxy)propyl]-4-piperidinyl]-6-fluoro-1,2-benzisoxazole as a beige solid, m.p. = 88-90°C.

| ANALYSIS: | | | |
|---|---|---|---|
| Calculated for C₂₂H₂₄BrFN₂O₃: | 57.03%C | 5.22%H | 6.05%N |
| Found: | 57.04%C | 5.19%H | 6.06%N |

### EXAMPLE 54

### 1-[4-[3-[4-(6-fluoro-1,2-benzisoxazol-3-yl)-1-piperidinyl]propoxy]-3-methoxyphenyl]propanone

A mixture of 6-fluoro-3-(4-piperidinyl)-1,2-benzisoxazole (2.8 g, 15.2 mmoles), K₂CO₃ (3 gm), 1-[4-(3-bromopropoxy)-3-methoxyphenyl]propanone (4.6 gm, 18.2 mmoles) in acetonitrile (100 ml) was heated at reflux for 2 hours. At the end of the reaction, the mixture was filtered and the solvent was concentrated and the residue was extracted into dichloromethane (300 ml). The dichloromethane was filtered and concentrated again. The crude material (6.4 g) was purified by flash chromatography over a silica gel column (SiO₂, 50 g; eluted with dichloromethane, 700 ml; 1% methanol in dichloromethane, 1.4 l). The material thus purified (weight: 2.87 g, 51%) was recrystallized from ethanol (25 ml) to give 2.13 g of 1-[4-[3-[4-(6-fluoro-1,2-benzisoxazol-3-yl)-1-piperidinyl]propoxy]-3-methoxyphenyl]propanone as beige colored crystals, m.p. = 118-119°C.

| ANALYSIS: | | | |
|---|---|---|---|
| Calculated for C₂₅H₂₉FN₂O₄: | 68.16%C | 6.64%H | 6.36%N |
| Found: | 68.32%C | 6.63%H | 6.29%N |

### EXAMPLE 55

### 4-[3-[4-(6-fluoro-1,2-benzisoxazol-3-yl)-1-piperidinyl]propoxy]-3-methoxybenzamide

A mixture of 6-fluoro-3-(4-piperidinyl)-1,2-benzisoxazole (2.2 g, 10.0 mmoles), K₂CO₃ (2.0 g) and 4-(3-bromopropoxy)-3-methoxybenzamide (2.32 g, 8.0 mmoles) in acetonitrile (80 ml) was heated at reflux for 5 hours. At the end of the reaction the solvent was evaporated. The residue was extracted into dichloromethane. The inorganic insolubles were filtered off. The dichloromethane was concentrated again. The crude residue was purified by flash chromatography over a silica gel column (55 gm, SiO₂; eluted with 1% methanol in dichloromethane, 1 l; 2% methanol in dichloromethane, 1 l). The material thus obtained weighed 2.93 g (84%) as white crystals. Recrystallization from hot ethanol (60 ml) gave 2.2 g of 4-[3-[4-(6-fluoro-1,2-benzisoxasol-3-yl)-1-piperidinyl]-propoxy]-3-methoxybenzamide as white crystals, m.p. = 163-164°C.

| ANALYSIS: | | | |
|---|---|---|---|
| Calculated for C₂₃H₂₆FN₃O₄: | 64.62%C | 6.13%H | 9.83%N |
| Found: | 64.20%C | 6.06%H | 9.71%N |

### EXAMPLE 56

### 1-[4-[3-[4-(6-Fluoro-1,2-benzisoxazol-3-yl)-1-piperidinyl]propoxy]-3-[methylamino)phenyl]ethanone

A mixture of 6-fluoro-3-(4-piperidinyl)-1,2-benzisoxazole (2.3 g, 0.0103 mol), K₂CO₃ (1.4 g, 0.0103 mol), 1-[4-(3-chloropropoxy)-3-(methylamino)phenyl]ethanone (2.5 g, 0.0103 mol), KI (0.10 g), and acetonitrile (100 ml) was stirred at reflux under nitrogen for 23 hours. The reaction was cooled to ambient temperature, poured into water, and the aqueous mixture was extracted with ethyl acetate. The ethyl acetate extract was washed twice with water, dried with MgSO₄ and was concentrated to yield 4.8 g of a damp, brown solid. The compound was isolated by preparative HPLC (®Waters Associates prep LC/System 500, utilizing 2 silica gel columns and 4% methanol-methylene chloride as eluent). Concentration of appropriate fractions afforded 2.4 g. Recrystallization from ethanol gave 2.1 g of 1-[4-[3-[4-(6-fluoro-1,2-benzisoxazol-3-yl)-1-piperidinyl]propoxy]-3-(methylamino)phenyl]ethanone as a beige solid, m.p. = 151-153°C.

| ANALYSIS: | | | |
|---|---|---|---|
| Calculated for C₂₄H₂₈FN₃O₃: | 67.75%C | 6.63%H | 9.88%N |
| Found: | 67.83%C | 6.76%H | 9.90%N |

### EXAMPLE 57

### 1-[4-[3-[4-(6-fluoro-1,2-benzisoxazol-3-yl-)-1-piperidinyl]propoxy]-3-ethoxyphenyl]ethanone

A suspension of NaH (0.28 g of a 50% oil dispersion, 0.0059 mol) in dimethylformamide (20 ml) was cooled to 4°C in an ice bath. To this was added, dropwise, 1-[4-[3-[4-(6-fluoro-1,2-benzisoxazol-3-yl)-1-piperidinyl]propoxy]-3-hydroxyphenyl]ethanone (2.3 g, 0.0056 mol) dissolved in dimethylformamide (40 ml). After total addition, the mixture was stirred under nitrogen for 1 hr. keeping the temperature below 10°C. A solution of bromoethane (1.3 g, 0.0118 mol) dissolved in dimethylformamide (15 ml) was then added, dropwise, to the reaction mixture. Stirring under nitrogen was continued for 3 hours allowing the temperature to slowly rise to ambient temperature. The reaction was cooled in an ice bath, water was added and the aqueous mixture was extracted with ethyl acetate. The ethyl acetate extract was washed with water, dried with MgSO₄, and was concentrated to yield 3.9 g of a damp, beige solid. The solid was triturated with diethyl ether and filtered to yield 1.5 g. This was combined with an additional sample (3.5 g total), and recrystallization from ethanol provided 3.0 g (57%) of glistening, beige crystals of 1-[4-[3-[4-(6-fluoro-1,2-benzisoxazol-3-yl)-1-piperidinyl]propoxy]-3-ethoxyphenyl]ethanone, m.p. = 112-114.

| ANALYSIS: | | | |
|---|---|---|---|
| Calculated for C₂₅H₂₉FN₂O₄: | 68.16%C | 6.64%H | 6.36%N |
| Found: | 68.10%C | 7.03%H | 6.35%N |

### EXAMPLE 58

### 1-[4-(3-Bromopropoxy)-3-(methylmercapto)phenyl]ethanone

A mixture of 1-[4-hydroxy-3-(methylmercapto)phenyl]ethanone (5.4 g; 0.03 mole), K₂CO₃ (4.2 g), 1,3-dibromopropane (8 g, 0.039 mole) in acetonitrile (150 ml) was heated at reflux for 3 hours and stirred at room temperature overnight. Acetonitrile was removed at reduced pressure and the residue was extracted into dichloromethane (250 ml). Insolubles were filtered off. The dichloromethane solution was concentrated. The crude product was purified on a silica gel column (SiO₂, 100 g; eluted with 3:2 hexane:dichloromethane, 1.6 l). The compound crystallized upon concentration, and the product (3.5 g, 39%) was recrystallized from ethanol (40 ml) to yield 1-[4-(3-bromopropoxy)-3-(methylmercapto)phenyl]ethanone as white needles, 2.0 g; m.p. = 120-122°C.

| ANALYSIS: | | |
|---|---|---|
| Calculated for C₁₂H₁₅BrO₂S: | 47.53%C | 4.99%H |
| Found: | 47.74%C | 4.91%H |

### EXAMPLE 59

### 4-(3-Bromopropoxy)-3-methoxybenzonitrile

A mixture of 4-hydroxy-3-methoxybenzonitrile (7.5 g, 50 mmoles), K₂CO₃ (12.5 g), and 1,3-dibromopropane (15 g, 75 mmoles) in acetonitrile (100 ml) was heated at reflux for 3 hours and left standing at room temperature overnight. The solvent of the reaction was removed on a rotary evaporator, and the crude solid was extracted into methylene chloride (500 ml). The insolubles were filtered off. The dichloromethane solution was concentrated and the material was purified on a flash chromatography column (SiO₂, 105 g; eluted with 2:3 dichloromethane:hexane, and then with dichloromethane). The desired product thus purified weighed 7.74 g (52%). Recrystallization twice from ethanol gave analytically pure 4-(3-bromopropoxy)-3-methoxybenzonitrile, m.p. = 99-101°C.

| ANALYSIS: | | | |
|---|---|---|---|
| Calculated for C₄H₁₂BrNO₂: | 48.91%C | 4.48%H | 5.19%N |
| Found: | 49.49%C | 4.47%H | 5.21%N |

### EXAMPLE 60

### 1-[4-(3-Bromopropoxy)-3-methylphenyl]ethanone

A mixture of 4-hydroxy-3-methylacetophenone (14.5 g 96 mmoles), K₂CO₃ (17.5 g, 144 mmoles), and 1,3-dibromopropane (30 g, 144 mmoles) in acetonitrile (400 ml) was heated at reflux for 6 hours. At the end of the reaction, the solvent was removed on a rotary evaporator, and the crude solid was extracted into dichloromethane (750 ml). The insoluble inorganics were filtered off. The dichloromethane solution was concentrated again to a crude oil (34.5 g). Purification was effected by flash chromatography over a silica gel column (SiO₂, 150 g; eluted with 7:3 hexane:dichloromethane, 2 l; and dichloromethane 2 l). The material thus purified weighed 14.6 gm (56%) and was recrystallized from ethanol. Recrystallization again from ethanol gave analytically pure 1-[4-(3-bromopropoxy)-3-methylphenyl]ethanone, m.p. = 59-61°C.

| ANALYSIS: | | |
|---|---|---|
| Calculated for C₁₂H₁₅BrO₂: | 53.15%C | 5.58%H |
| Found: | 53.35%C | 5.52%H |

### EXAMPLE 61

### 1-[4-(3-Bromopropoxy)-3-methoxyphenyl]phenylmethanone

A mixture of 1-(4-hydroxy-3-methoxyphenyl)phenylmethanone (14 g, 61.4 mmoles), K₂CO₃ (13 g, 92.1 mmoles), and 1,3-dibromopropane (28 g, 86 mmoles) in acetonitrile (400 ml) was heated at reflux for 4 hours. The reaction was followed by thin layer chromatography. At the end of the reaction, the inorganics were filtered off and the solvent was removed on a rotary evaporator. The residue was purified on a flash chromatographic column (SiO₂, 140 g, eluted with 4:1 hexane:dichloromethane, 1.2 l) to give a partially solidified material: 15.44 g (72%). Recrystallization twice from ethanol gave 2.84 g of 1-[4-(3-bromopropoxy)-3-methoxyphenyl]phenylmethanone as white crystals, m.p. = 88-89°C.

| ANALYSIS: | | |
|---|---|---|
| Calculated for C₁₇H₁₇BrO₃: | 58.47%C | 4.91%H |
| Found: | 59.03%C | 4.87%H |

This invention thus provides a group of chemical compounds that are capable of producing antipsychotic effects and may be capable of affecting negative symptoms of schizophrenia in a beneficial manner. In addition, many of the compounds may also have reduced tendencies to produce extrapyramidal side effects in mammals.

## Claims (Claims for the following Contracting State(s): AT, BE, CH, DE, DK, FR, GB, IT, LI, LU, NL, SE)

1. A compound of the formula: 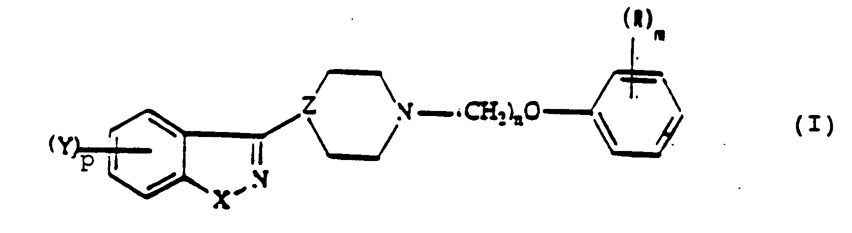 wherein X is -O-, -S-, -NH-, or 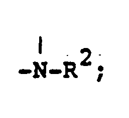 Y is hydrogen, C₁-C₆-alkyl, -OH, Cl, F, BR, I, C₁-C₆-alkoxy, -CF₃- NO₂, or -NH₂, when p is 1;
Y is C₁ ― C₆-alkoxy when p is 2 and X is -O-;
R² is selected from the group consisting of lower alkyl, aryl lower alkyl, aryl, cycloalkyl, aroyl, alkanoyl, and phenyl sulfonyl groups;
Z is 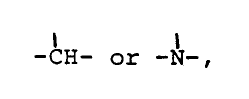 with the proviso that Z is not 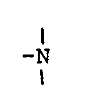 when X is -S- and R is H
n is 2, 3, 4, or 5;
R is hydrogen, alkyl, C₁ - C₆ alkoxy, hydroxyl, carboxyl, Cl, F, Br, I, amino, C₁ - C₆ mono or dialkyl amino, -NO₂, lower alkyl thio, -OCF₃, cyano, acylamino, -CF₃, trifluoroacetyl, aminocarbonyl, 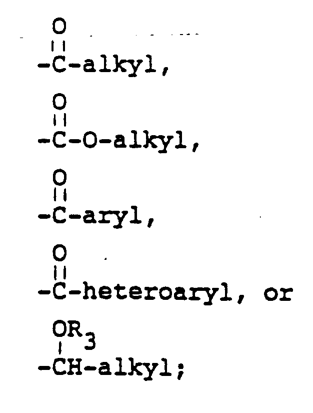 alkyl is lower alkyl;
aryl is phenyl or 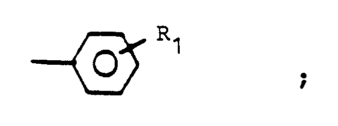 where R₁ is hydrogen, lower alkyl, C₁ - C₆ alkoxy, hydroxy, Cl, F, Br, I, C₁ - C₆ alkyl amino, -NO₂, -CN, -CF₃, -OCF₃;
heteroaryl is 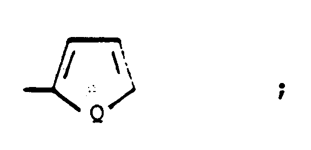 Q is -O-, -S-, 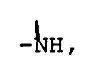 -CH=N-;
m is 1, 2 or 3, with the proviso that when m is 2 or 3, R can be the same or different;
R₃ is hydrogen, lower alkyl, or acyl;
or a pharmaceutically acceptable acid addition salt thereof.

2. A compound as claimed in claim 1, wherein X is -O-, -S- or -NH.

3. A compound as claimed in claim 2, wherein n is 2, 3 or 4,, Y is hydrogen or is chlorine or fluorine in the 6-position and p is 1.

4. A compound as claimed in claim 3, wherein R is hydrogen, C₁-C₆-alkyl, C₁-C₆-alkoxy, C₁-C₆-alkylthio, halogen, C₁-C₆-alkylamino 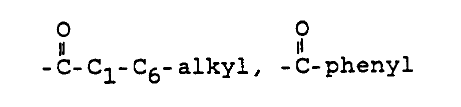 or -CH(OR₃)-C₁-C₆-alkyl and m is 2 or 3.

5. A compound as claimed in claim 3, wherein n is 3 or 4 m is 2, and R is -OCH₃ and

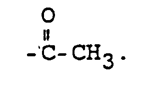

6. A compound as claimed in claim 1, which is 1-[4-[3-[4-(1H-indazol-3-yl)-1-piperazinyl]propoxy]-3-methoxyphenyl]ethanone or a pharmaceutically acceptable acid addition salt thereof.

7. A compound as claimed in claim 1, which is 1-[4-[3-[4-(1,2-benzisoxazol-3-yl)-1-piperidinyl]propoxy]-3-methoxyphenyl)ethanone or a pharmaceutically acceptable acid addition salt thereof.

8. A compound as claimed in claim 1 which is 1-[4-[3-[4-(6-fluoro-1,2-benzisoxazol-3-yl)-1-piperidinyl)propoxy]-3-methoxyphenyl]ethanone or a pharmaceutically acceptable acid addtion salt thereof.

9. A compound as claimed in claim 1, which is 1-[4-[4-[4-(1,2-benzisoxazol-3-yl)-1-piperidinyl]butoxy]-3-methoxyphenyl]ethanone or a pharmaceutically acceptable acid addition salt thereof.

10. A compound as claimed in claim 1, which is 1-[4-[4-[4-(6-fluoro-1,2-benzisoxazol-3-yl)-1-piperidinyl]butoxy]- 3-methoxyphenyl]ethanone or a pharmaceutically acceptable acid addition salt thereof.

11. A pharmaceutical composition, which comprises as the action ingredient a compound as claimed in claim 1 and a suitable carrier therefor.

12. Use of a compound as claimed in claim 1 for the preparation of a medicament having either antipsychotic or antipsychotic and analgesic activity.

13. A process for the preparation of a compound as claimed in claim 1, which comprises
a) reacting a compound of the formula II 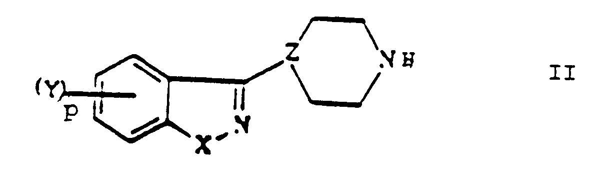 wherein X, Y, Z and p are as defined, under alkylating conditions with a compound of the formula III 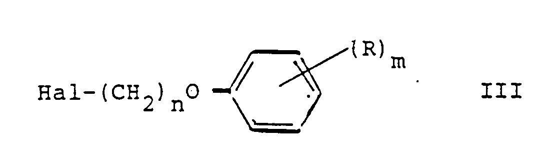 wherein R, m and n are as defined and Hal is chlorine, bromine or iodine,
b) optionally reducing a compound of the formula I, wherein R is 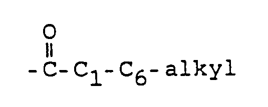 to obtain a compound of the formula I, wherein R is CH₂(OH)-C₁-C₆-alkyl,
c) optionally reacting a compound of the formula I, wherein R is -CH(OR₃)-C₁-C₆-alkyl, where R₃ is hydrogen, with an alcanoic acid anhydride to obtain a compound of the formula I, wherein R is C₁-C₆-alkanoyl,
d) optionally reacting a compound of the formula I, wherein R is hydroxy, first with a strong base and then with a compound of the formula Hal-C₁-C₆-alkyl, where Hal is chlorine, bromine or iodine, to obtain a compound of the formula I, where R is C₁-C₆-alkoxy,
e) optionally reacting a compound of the formula I, wherein X is N-R₂, and R₂ is hydrogen, with a C₁-C₆-alkanoyl or aroyl halide to obtain a compound of the formula I, wherein X is NR₂, and R₂ is C₁-C₆-alkanoyl or aroyl.

14. A process as claimed in claim 14 wherein the reaction of step a) is carried out in an inert solvent at a temperature of from 50°C to the reflux temperature of the solvent in the presence of an acid receptor.

## Claims (Claims for the following Contracting State(s): ES, GR)

1. A process for the preparation of a compound of the formula I 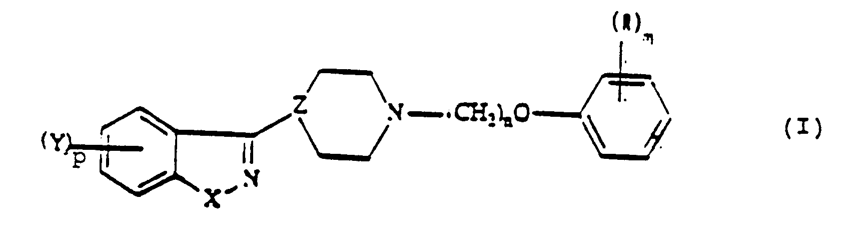 wherein X is -O-, -S-, -NH-, or 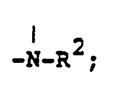 Y is hydrogen, C₁-C₆-alkyl, -OH, Cl, F, BR, I, C₁-C₆-alkoxy, -CF₃- NO₂, or -NH₂, when p is 1;
Y is C₁ ― C₆-alkoxy when p is 2 and X is -O-;
R² is selected from the group consisting of lower alkyl, aryl lower alkyl, aryl, cycloalkyl, aroyl, alkanoyl, and phenyl sulfonyl groups;
Z is 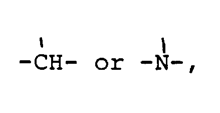 with the proviso that Z is not 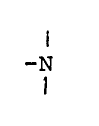 when X is -S- and R is H
n is 2, 3, 4, or 5;
R is hydrogen, alkyl, C₁ - C₆ alkoxy, hydroxyl, carboxyl, Cl, F, Br, I, amino, C₁ - C₆ mono or dialkyl amino, -NO₂, lower alkyl thio, -OCF₃, cyano, acylamino, -CF₃, trifluoroacetyl, aminocarbonyl, 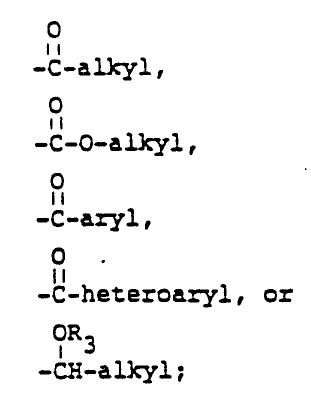 alkyl is lower alkyl;
aryl is phenyl or 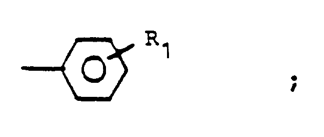 where R₁ is hydrogen, lower alkyl, C₁ - C₆ alkoxy, hydroxy, Cl, F, Br, I, C₁ - C₆ alkyl amino, -NO₂, -CN, -CF₃, -OCF₃;
heteroaryl is 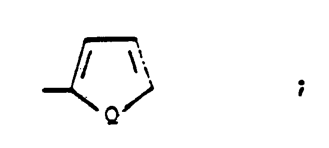 Q is -O-, -S-, -NH, -CH=N-;
m is 1, 2 or 3, with the proviso that when m is 2 or 3, R can be the same or different; and R₃ is hydrogen, lower alkyl, or acyl;
or a pharmaceutically acceptable acid addition salt thereof. which comprises
a) reacting a compound of the formula II 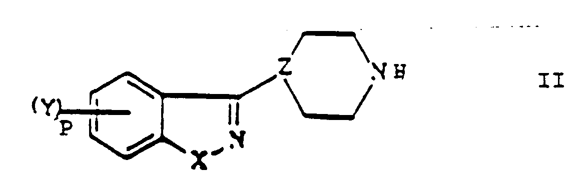 wherein X, Y, Z and p are as defined, under alkylating conditions with a compound of the formula III 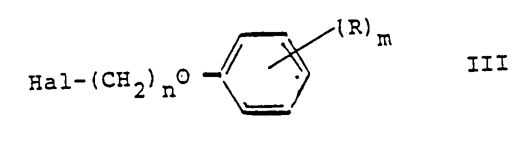 wherein R, m and n are as defined and Hal is chlorine, bromine or iodine,
b) optionally reducing a compound of the formula I, wherein R is 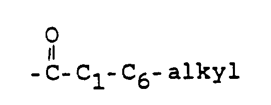 to obtain a compound of the formula I, wherein R is CH₂(OH)-C₁-C₆-alkyl,
c) optionally reacting a compound of the formula I, wherein R is -CH(OR₃)-C₁-C₆-alkyl, where R₃ is hydrogen, with an alcanoic acid anhydride to obtain a compound of the formula I, wherein R is C₁-C₆-alkanoyl,
d) optionally reacting a compound of the formula I, wherein R is hydroxy, first with a strong base and then with a compound of the formula Hal-C₁-C₆-alkyl, where Hal is chlorine, bromine or iodine, to obtain a compound of the formula I, where R is C₁-C₆-alkoxy,
e) optionally reacting a compound of the formula I, wherein X is N-R₂, and R₂ is hydrogen, with a C₁-C₆-alkanoyl or aroyl halide to obtain a compound of the formula I, wherein X is NR₂, and R₂ is C₁-C₆-alkanoyl or aroyl.

2. A process as claimed in claim 1, wherein the reaction of step a) is carried out in an inert solvent at a temperature of from 50°C to the reflux temperature of the solvent in the presence of an acid receptor.

3. A process as claimed in claim 2 wherein an alkali metal carbonate is used as the acid receptor.

4. A process as claimed in claim 1, wherein the reduction according to step b) is carried out by using sodium borohydride.

5. A process as claimed in claim 1, wherein the strong base used in step d) is sodium hydride.

6. A process as claimed in claim 1, wherein X is -O-, -S- or -NH.

7. A process as claimed in claim 1 wherein n is 2, 3 or 4, Y is hydrogen or is chlorine or fluorine in the 6-position and p is 1.

8. A process as claimed in claim 1, wherein R is hydrogen, halogen C₁-C₆-alkyl, C₁-C₆-alkoxy, C₁-C₆-alkylthio, C₁-C₆-alkylamino, 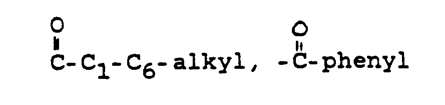 or -CH(OR₃)-C₁-C₆-alkyl where R₃ is hydrogen or C₁-C₆-alkyl and m is 2 or 3.

9. A process as claimed in claim 1, wherein n is 3 or 4, m is 2 and R is -OCH₃ and

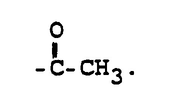

10. A process as claimed in claim 1, wherein 1-[4-[3-[4-(1H-indazol-3-yl)-1-piperazinyl]propoxy]-3-methoxyphenyl]ethanone or a pharmaceutically acceptable acid addition salt thereof is prepared.

11. A process as claimed in claim 1, wherein 1-[4-[3-[4-(1,2-benzisoxazol-3-yl)-1-piperidinyl]propoxy]3-methoxyphenyl)ethanone or a pharmaceutically acceptable acid addition salt thereof is prepared.

12. A process as claimed in claim 1 wherein 1-[4-[3-[4-(6-fluoro-1,2-benzisoxazol-3-yl)-1-piperidinyl)propoxy]-3-methoxyphenyl]ethanone or a pharmaceutically acceptable acid addtion salt thereof is prepared.

13. A process as claimed in claim 1, wherein 1-[4-[4-[4-(1,2-benzisoxazol-3-yl)-1-piperidinyl]butoxy]3-methoxyphenyl]ethanone or a pharmaceutically acceptable acid addition salt thereof is prepared.

14. A process as claimed in claim 1, wherein 1-[4-[4-[4-(6-fluoro-1,2-benzisoxazol-3-yl)-1-piperidinyl]butoxy]-3-methoxyphenyl]ethanone or a pharmaceutically acceptable acid addition salt thereof is prepared.

15. Use of a compound of the formula I as defined in claim 1 for the preparation of a medicament having either antipsychotic or antipsychotic and analgesic activity.

## Patentansprüche (Patentansprüche für folgende(n) Vertragsstaat(en): AT, BE, CH, DE, DK, FR, GB, IT, LI, LU, NL, SE)

1. Verbindung der Formel: 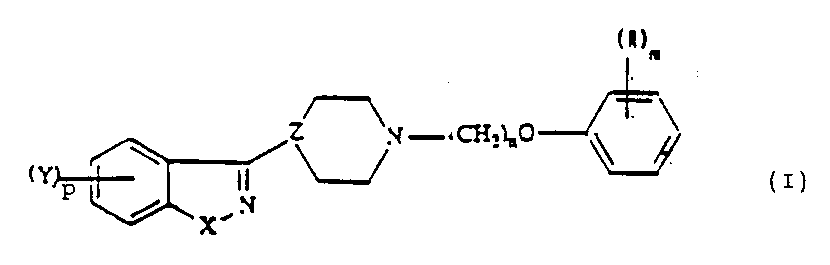 in welcher
X für -O-, -S-, -NH- oder 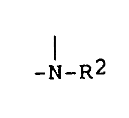 steht;
Y für Wasserstoff, C₁-C₆-Alkyl, -OH, Cl, F, Br, I, C₁-C₆-Alkoxy, -CF₃, -NO₂ oder -NH₂ steht, wenn p 1 ist;
Y für C₁-C₆-Alkoxy steht, wenn p 2 ist und X -O- ist;
R² aus der aus Niederalkyl-, Arylniederalkyl-, Aryl-, Cycloalkyl-, Aroyl-, Alkanoyl- und Phenylsulfonylgruppen bestehenden Gruppe ausgewählt wird;
Z für 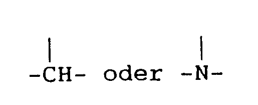 steht, unter dem Vorbehalt, daß Z nicht 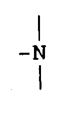 ist, wenn X für -S- und R für H steht;
n für 2, 3, 4 oder 5 steht;
R für Wasserstoff, Alkyl, C₁-C₆-Alkoxy, Hydroxyl, Carboxyl, Cl, F, Br, I, Amino, C₁-C₆-Mono- oder Dialkylamino, -NO₂, Niederalkylthio, -OCF₃, Cyano, Acylamino, -CF₃, Trifluoracetyl, Aminocarbonyl, 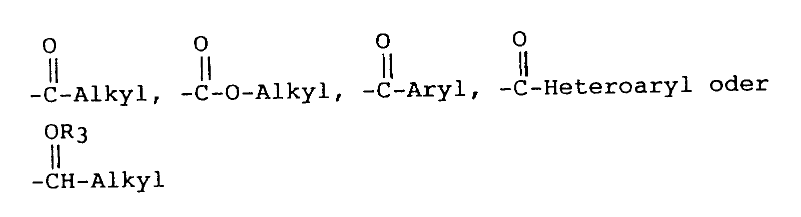 steht;
Alkyl Niederalkyl bedeutet;
Aryl Phenyl oder 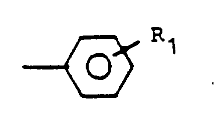 darstellt, wobei R₁ für Wasserstoff, Niederalkyl, C₁-C₆-Alkoxy, Hydroxy, Cl, F, Br, I, C₁-C₆-Alkylamino, -NO₂, -CN, -CF₃, -OCF₃ steht; Heteroaryl für 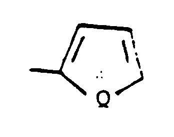 steht, wobei Q -O-, -S-, 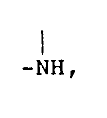 -CH=N- bedeutet;
m für 1, 2 oder 3 steht, unter der Voraussetzung daß R dieselbe oder eine unterschiedliche Bedeutung haben kann, wenn m für 2 oder 3 steht;
R₃ für Wasserstoff, Niederalkyl oder Acyl steht;
oder ein pharmazeutisch verträgliches Säureadditionssalz davon.

2. Verbindung gemäß Anspruch 1, in welcher X für -O-, -S- oder -NH steht.

3. Verbindung gemäß Anspruch 2, in welcher n für 2, 3 oder 4 steht, Y für Wasserstoff oder für Chlor oder Fluor in Position 6 und p für 1 steht.

4. Verbindung gemäß Anspruch 3, in welcher R für Wasserstoff, C₁-C₆-Alkyl, C₁-C₆-Alkoxy, C₁-C₆-Alkylthio, Halogen, C₁-C₆-Alkylamino, 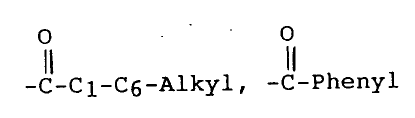 oder -CH(OR₃)-C₁-C₆-Alkyl und m für 2 oder 3 steht.

5. Verbindung gemäß Anspruch 3, in welcher n für 3 oder 4, m für 2 und R für -OCH₃ und 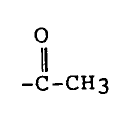 steht.

6. Verbindung gemäß Anspruch 1, bei der es sich um 1-[4-[3-[4-(1H-Indazol-3-yl)-1-piperazinyl]propoxy]-3-methoxyphenyl]ethanon oder ein pharmazeutisch verträgliches Säureadditionssalz davon handelt.

7. Verbindung gemäß Anspruch 1, bei der es sich um 1-[4-[3-[4-(1,2-Benzisoxazol-3-yl)-1-piperidinyl]propoxy]-3-methoxyphenyl]ethanon oder ein pharmazeutisch verträgliches Säureadditionssalz davon handelt.

8. Verbindung gemäß Anspruch 1, bei der es sich um 1-[4-[3-[4-(6-Fluor-1,2-benzisoxazol-3-yl)-1-piperidinyl]propoxy]-3-methoxyphenyl]ethanon oder ein pharmazeutisch verträgliches Säureadditionssalz davon handelt.

9. Verbindung gemäß Anspruch 1, bei der es sich um 1-[4-[4-[4-(1,2-Benzisoxazol-3-yl)-1-piperidinyl]butoxy]-3-methoxyphenyl]ethanon oder ein pharmazeutisch verträgliches Säureadditionssalz davon handelt.

10. Verbindung gemäß Anspruch 1, bei der es sich um 1-[4-[4-[4-(6-Fluor-1,2-benzisoxazol-3-yl)-1-piperidinyl]butoxy]-3-methoxyphenyl]ethanon oder ein pharmazeutisch verträgliches Säureadditionssalz davon handelt.

11. Pharmazeutische Zusammensetzung, die als Wirkstoff eine Verbindung gemäß Anspruch 1 und eine geeignete Trägersubstanz dafür enthält.

12. Verwendung einer Verbindung gemäß Anspruch 1 zur Herstellung eines Medikaments mit antipsychotischer Wirksamkeit oder mit antipsychotischer und analgetischer Wirksamkeit.

13. Verfahren zur Herstellung einer Verbindung gemäß Anspruch 1, das folgende Schritte umfaßt:
a) Umsetzen einer Verbindung der Formel II 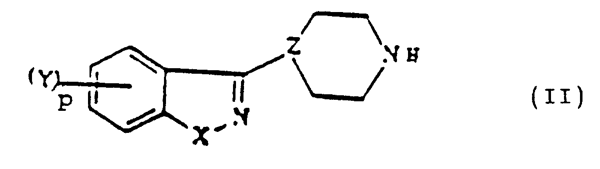 in welcher X, Y, Z und p die weiter oben angewiesene Bedeutung zukommt, unter Alkylierungsbedingungen mit einer Verbindung der Formel III 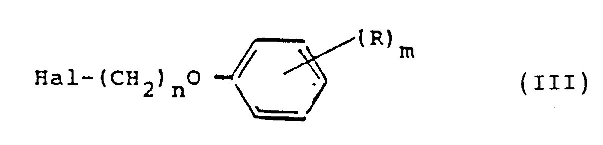 in welcher R, m und n wie vorstehend angegeben definiert sind, und Hal für Chlor, Brom oder Jod steht,
b) wahlweise Reduzieren einer Verbindung der Formel I, in welcher R für 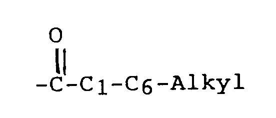 steht, zur Bildung einer Verbindung der Formel I, in welcher R CH₂(OH)-C₁-C₆-Alkyl bedeutet,
c) wahlweise Umsetzen einer Verbindung der Formel I, in welcher R -CH(OR₃)-C₁-C₆-Alkyl bedeutet, wobei R₃ Wasserstoff ist, mit einem Alkanoesäureanhydrid zur Bildung einer Verbindung der Formel I, in welcher R C₁-C₆-Alkanoyl ist,
d) wahlweise Umsetzen einer Verbindung der Formel I, in welcher R Hydroxy bedeutet, zunächst mit einer starken Base und dann mit einer Verbindung der Formel Hal-C₁-C₆-alkyl, in welcher Hal für Chlor, Brom oder Jod steht, zur Bildung einer Verbindung der Formel I, in welcher R C₁-C₆-Alkoxy ist,
e) wahlweise Umsetzen einer Verbindung der Formel I, in welcher X N-R₂ ist und R₂ für Wasserstoff steht, mit einem C₁-C₆-Alkanoyl- oder Aroylhalogenid zur Bildung einer Verbindung der Formel I, in welcher X für NR₂ und R₂ für C₁-C₆-Alkanoyl oder Aroyl steht.

14. Verfahren gemäß Anspruch 13, bei dem die Umsetzung von Schritt a) in einem inerten Lösemittel bei einer Temperatur zwischen 50°C und der Rückflußtemperatur des Lösemittels in Gegenwart eines Säureakzeptors erfolgt.

## Patentansprüche (Patentansprüche für folgende(n) Vertragsstaat(en): ES, GR)

1. Verfahren zur Herstellung einer Verbindung der Formel I 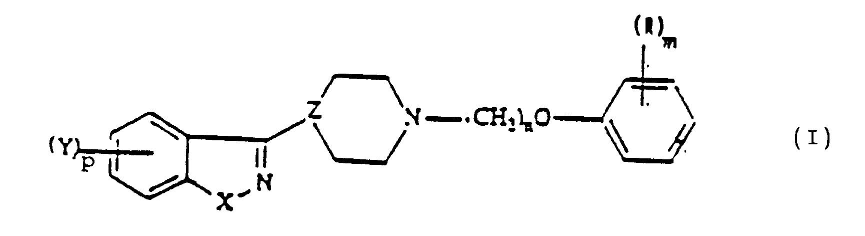 in welcher
X für -O-, -S-, -NH- oder 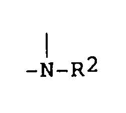 steht;
Y für Wasserstoff, C₁-C₆-Alkyl, -OH, Cl, F, Br, I, C₁-C₆-Alkoxy, -CF₃, -NO₂ oder -NH₂ steht, wenn p 1 ist;
Y für C₁-C₆-Alkoxy steht, wenn p 2 ist und X -O- ist;
R₂ aus der aus Niederalkyl-, Arylniederalkyl-, Aryl-, Cycloalkyl-, Aroyl-, Alkanoyl- und Phenylsulfonylgruppen bestehenden Gruppe ausgewählt wird;
Z für 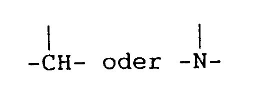 steht, unter dem Vorbehalt, daß Z nicht 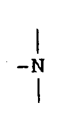 ist, wenn X für -S- und R für H steht;
n für 2, 3, 4 oder 5 steht;
R für Wasserstoff, Alkyl, C₁-C₆-Alkoxy, Hydroxyl, Carboxyl, Cl, F, Br, I, Amino, C₁-C₆-Mono- oder Dialkylamino, -NO₂, Niederalkylthio, -OCF₃, Cyano, Acylamino, -CF₃, Trifluoracetyl, Aminocarbonyl, 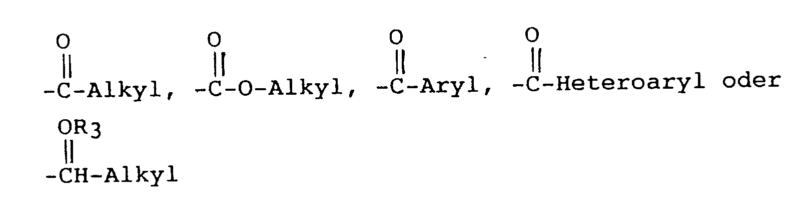 steht;
Alkyl Niederalkyl bedeutet;
Aryl Phenyl oder 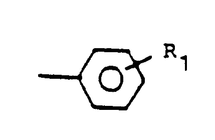 darstellt, wobei R₁ für Wasserstoff, Niederalkyl, C₁-C₆-Alkoxy, Hydroxy, Cl, F, Br, I, C₁-C₆-Alkylamino, -NO₂, -CN, -CF₃, -OCF₃ steht; Heteroaryl für 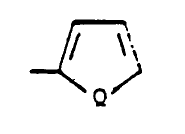 steht, wobei Q -O-, -S-, 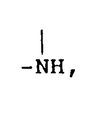 -CH=N- bedeutet;
m für 1, 2 oder 3 steht, unter der Voraussetzung daß R dieselbe oder eine unterschiedliche Bedeutung haben kann, wenn m für 2 oder 3 steht;
R₃ für Wasserstoff, Niederalkyl oder Acyl steht;
oder eines pharmazeutisch verträglichen Säureadditionssalzes davon,
das folgende Schritte umfaßt:
a) Umsetzen einer Verbindung der Formel II 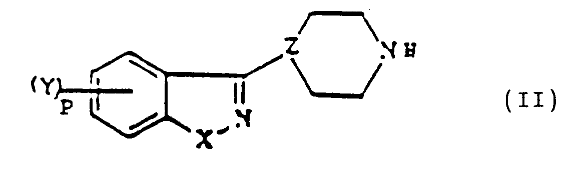 in welcher X, Y, Z und p die weiter oben angewiesene Bedeutung zukommt, unter Alkylierungsbedingungen mit einer Verbindung der Formel III 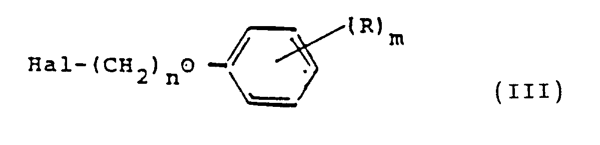 in welcher R, m und n wie vorstehend angegeben definiert sind, und Hal für Chlor, Brom oder Jod steht,
b) wahlweise Reduzieren einer Verbindung der Formel I, in welcher R für 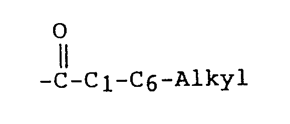 steht, zur Bildung einer Verbindung der Formel I, in welcher R CH₂(OH)-C₁-C₆-Alkyl bedeutet,
c) wahlweise Umsetzen einer Verbindung der Formel I, in welcher R -CH(OR₃)-C₁-C₆-Alkyl bedeutet, wobei R₃ Wasserstoff ist, mit einem Alkanoesäureanhydrid zur Bildung einer Verbindung der Formel I, in welcher R C₁-C₆-Alkanoyl ist,
d) wahlweise Umsetzen einer Verbindung der Formel I, in welcher R Hydroxy bedeutet, zunächst mit einer starken Base und dann mit einer Verbindung der Formel Hal-C₁-C₆-alkyl, in welcher Hal für Chlor, Brom oder Jod steht, zur Bildung einer Verbindung der Formel I, in welcher R C₁-C₆-Alkoxy ist,
e) wahlweise Umsetzen einer Verbindung der Formel I, in welcher X N-R₂ ist und R₂ für Wasserstoff steht, mit einem C₁-C₆-Alkanoyl- oder Aroylhalogenid zur Bildung einer Verbindung der Formel I, in welcher X für NR₂ und R₂ für C₁-C₆-Alkanoyl oder Aroyl steht.

2. Verfahren gemäß Anspruch 1, bei dem die Umsetzung von Schritt a) in einem inerten Lösemittel bei einer Temperatur zwischen 50°C und der Rückflußtemperatur des Lösemittels in Gegenwart eines Säureakzeptors erfolgt.

3. Verfahren gemäß Anspruch 2, wobei ein Alikalimetallcarbonat als Säureakzeptor verwendet wird.

4. Verfahren gemäß Anspruch 1, wobei die in Schritt b) dargestellte Reduktionsreaktion unter Verwendung von Natriumborhydrid durchgeführt wird.

5. Verfahren gemäß Anspruch 1, wobei die in Schritt d) eingesetzte starke Base Natriumhydrid ist.

6. Verfahren gemäß Anspruch 1, wobei X für -O-, -S- oder -NH steht.

7. Verfahren gemäß Anspruch 1, wobei n für 2, 3 oder 4 steht, Y Wasserstoff, Chlor oder Fluor in der Position 6 darstellt und p für 1 steht.

8. Verfahren gemäß Anspruch 1, wobei R für Wasserstoff, Halogen, C₁-C₆-Alkyl, C₁-C₆-Alkoxy, C₁-C₆-Alkylthio, C₁-C₆-Alkylamino, 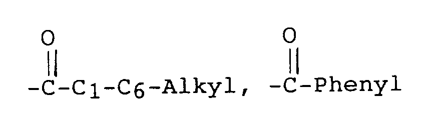 oder -CH(OR₃)-C₁-C₆-Alkyl und m für 2 oder 3 steht.

9. Verfahren gemäß Anspruch 1, wobei n für 3 oder 4, m für 2 und R für -OCH₃ und 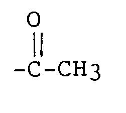 steht.

10. Verfahren gemäß Anspruch 1, bei dem 1-[4-[3-[4-(1H-Indazol-3-yl)-1-piperazinyl]propoxy]-3-methoxyphenyl]ethanon oder ein pharmazeutisch verträgliches Säureadditionssalz davon hergestellt wird.

11. Verfahren gemäß Anspruch 1, bei dem 1-[4-[3-[4-(1,2-Benzisoxazol-3-yl)-1-piperidinyl]propoxy]-3-methoxyphenyl]ethanon oder ein pharmazeutisch verträgliches Säureadditionssalz davon hergestellt wird.

12. Verfahren gemäß Anspruch 1, bei dem 1-[4-[3-[4-(6-Fluor-1,2-benzisoxazol-3-yl)-1-piperidinyl]propoxy]-3-methoxyphenyl]ethanon oder ein pharmazeutisch verträgliches Säureadditionssalz davon hergestellt wird.

13. Verfahren gemäß Anspruch 1, bei dem 1-[4-[4-[4-(1,2-Benzisoxazol-3-yl)-1-piperidinyl]butoxy]-3-methoxyphenyl]ethanon oder ein pharmazeutisch verträgliches Säureadditionssalz davon hergestellt wird.

14. Verfahren gemäß Anspruch 1, bei dem 1-[4-[4-[4-(6-Fluor-1,2-benzisoxazol-3-yl)-1-piperidinyl]butoxy]-3-methoxyphenyl]ethanon oder ein pharmazeutisch verträgliches Säureadditionssalz davon hergestellt wird.

15. Verwendung einer Verbindung der Formel I gemäß Anspruch 1 zur Herstellung eines Medikaments mit antipsychotischer Wirksamkeit oder mit antipsychotischer und analgetischer Wirksamkeit.

## Revendications (Revendications pour l'(les) Etat(s) contractant(s) suivant(s): AT, BE, CH, DE, DK, FR, GB, IT, LI, LU, NL, SE)

1. Composé de formule 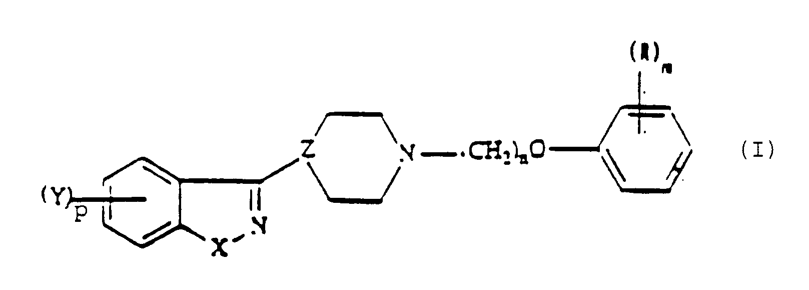 dans laquelle
X est -O-, -S-, -NH- ou 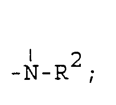
Y est un atome d'hydrogène ou un groupe alkyle en C₁-C₆, -OH, Cl, F, Br, I, alcoxy en C₁-C₆, -CF₃, -NO₂ ou -NH₂, lorsque p est 1;
Y est un groupe alcoxy en C₁-C₆ lorsque p est 2 et X est -O-;
R² est choisi parmi des groupes alkyle inférieur, arylalkyle inférieur, aryle, cycloalkyle, aroyle, alcanoyle et phénylsulfonyle;
Z est 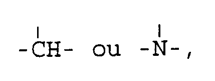 étant entendu que Z n'est pas 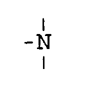 lorsque X est -S- et R est H;
n est 2, 3, 4 ou 5;
R est un atome d'hydrogène ou un groupe alkyle, alcoxy en C₁-C₆, hydroxy, carboxy, Cl, F, Br, I, un groupe amino, mono- ou dialkyl(C₁-C₆)amino, -NO₂, alkylthio inférieur, -OCF₃, cyano, acylamino, -CF₃, trifluoroacétyle, aminocarbonyle, 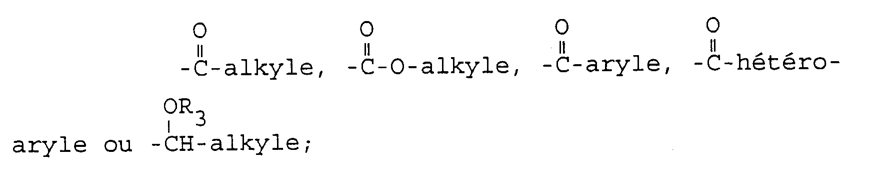
le groupe alkyle est un groupe alkyle inférieur;
le groupe aryle est le groupe phényle ou un groupe 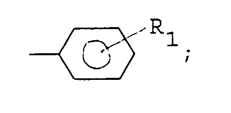 dans lequel R₁ est un atome d'hydrogène ou un groupe alkyle inférieur, alcoxy en C₁-C₆, hydroxy, Cl, F, Br, I, un groupe alkyl(C₁-C₆)amino, -NO₂, -CN, -CF₃, -OCF₃;
un groupe hétéroaryle est un groupe 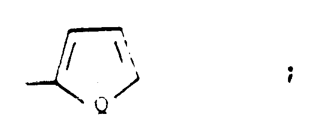
Q est -O-, -S-, 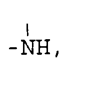 -CH=N-;
m est 1, 2 ou 3, étant entendu que lorsque m est 2 ou 3, les radicaux R peuvent être identiques ou différents;
R₃ est un atome d'hydrogène ou un groupe alkyle inférieur ou acyle;
ou sel d'addition avec un acide pharmaceutiquement acceptable de celui-ci.

2. Composé selon la revendication 1, dans lequel X est -O-, -S- ou -NH-.

3. Composé selon la revendication 2, dans lequel n est 2, 3 ou 4, Y est un atome d'hydrogène, de chlore ou de fluor en position 6 et p est 1.

4. Composé selon la revendication 3, dans lequel R est un atome d'hydrogène ou d'halogène ou un groupe alkyle en C₁-C₆, alcoxy en C₁-C₆, alkyl(C₁-C₆)-thio, alkyl(C₁-C₆)-amino, 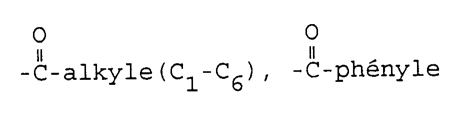 ou -CH(OR₃)-alkyle-(C₁-C₆) et m est 2 ou 3.

5. Composé selon la revendication 3, dans lequel n est 3 ou 4, m est 2 et les radicaux R sont -OCH₃ et

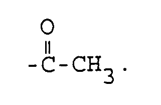

6. Composé selon la revendication 1, qui est la 1-[4-[3-[4-(1H indazol-3-yl)-1-pipérazinyl]propoxy]-3-méthoxyphényl]éthanone ou un sel d'addition avec un acide pharmaceutiquement acceptable de celle-ci.

7. Composé selon la revendication 1, qui est la 1-[4-[3-[4-(1,2-benzisoxazol-3-yl)-1-pipéridinyl]propoxy]-3-méthoxyphényl]éthanone ou un sel d'addition avec un acide pharmaceutiquement acceptable de celle-ci.

8. Composé selon la revendication 1, qui est la 1-[4-[3-[4-(6-fluoro-1,2-benzisoxazol-3-yl)-1-pipéridinyl]propoxy]-3-méthoxyphényl]éthanone ou un sel d'addition avec un acide pharmaceutiquement acceptable de celle-ci.

9. Composé selon la revendication 1, qui est la 1-[4-[4-[4-(1,2-benzisoxazol-3-yl)-1-pipéridinyl]butoxy]-3-méthoxyphényl]éthanone ou un sel d'addition avec un acide pharmaceutiquement acceptable de celle-ci.

10. Composé selon la revendication 1, qui est la 1-[4-[4-[4-(6-fluoro-1,2-benzisoxazol-3-yl)-1-pipéridinyl]butoxy]-3-méthoxyphényl]éthanone ou un sel d'addition avec un acide pharmaceutiquement acceptable de celle-ci.

11. Composition pharmaceutique comprenant, en tant que composant actif, un composé selon la revendication 1 et un véhicule approprié pour celui-ci.

12. Utilisation d'un composé selon la revendication 1, pour la fabrication d'un médicament ayant une activité soit antipsychotique, soit antipsychotique et analgésique.

13. Procédé pour la préparation d'un composé selon la revendication 1, comprenant
a) la mise en réaction d'un composé de formule II 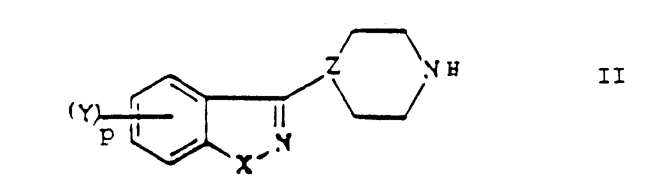 dans laquelle X, Y, Z et p sont tels que définis, dans des conditions d'alkylation, avec un composé de formule III 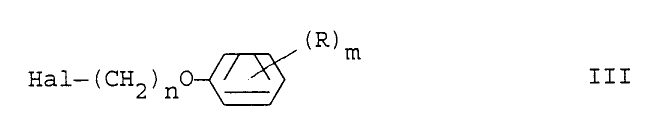 dans laquelle R, m et n sont tels que définis, et Hal est un atome de chlore, de brome ou d'iode,
b) éventuellement la réduction d'un composé de formule I dans lequel R est un groupe 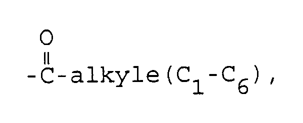 pour l'obtention d'un composé de formule I dans lequel R est un groupe CH₂(OH)-alkyle(C₁-C₆),
c) éventuellement la mise en réaction d'un composé de formule I dans lequel R est un groupe -CH(OR₃)-alkyle(C₁-C₆), dans lequel R₃ est un atome d'hydrogène, avec un anhydride d'acide alcanoïque, pour l'obtention d'un composé de formule I dans lequel R est un groupe alcanoyle en C₁-C₆,
d) éventuellement la mise en réaction d'un composé de formule I dans lequel R est le groupe hydroxy, d'abord avec une base forte et ensuite avec un composé de formule Hal-alkyle(C₁-C₆), dans lequel Hal est un atome de chlore, de brome ou d'iode, pour l'obtention d'un composé de formule I dans lequel R est un groupe alcoxy en C₁-C₆,
e) éventuellement la mise en réaction d'un composé de formule I dans lequel X est N-R₂, et R₂ est un atome d'hydrogène, avec un halogénure d'alcanoyle en C₁-C₆ ou d'aroyle, pour l'obtention d'un composé de formule I dans lequel X est NR₂, et R₂ est un groupe alcanoyle en C₁-C₆ ou aroyle.

14. Procédé selon la revendication 13, dans lequel la réaction de l'étape a) est effectuée dans un solvant inerte, à une température dans la plage allant de 50°C à la température de reflux du solvant, en présence d'un capteur d'acide.

## Revendications (Revendications pour l'(les) Etat(s) contractant(s) suivant(s): ES, GR)

1. Procédé pour la préparation d'un composé de formule I 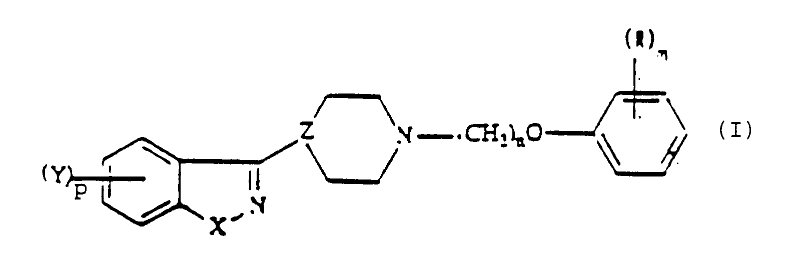 dans laquelle
X est -O-, -S-, -NH- ou 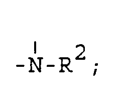
Y est un atome d'hydrogène ou un groupe alkyle en C₁-C₆, -OH, Cl, F, Br, I, alcoxy en C₁-C₆, -CF₃, -NO₂ ou -NH₂, lorsque p est 1;
Y est un groupe alcoxy en C₁-C₆ lorsque p est 2 et X est -O-;
R² est choisi parmi des groupes alkyle inférieur, arylalkyle inférieur, aryle, cycloalkyle, aroyle, alcanoyle et phénylsulfonyle,
Z est 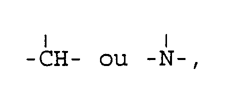 étant entendu que Z n'est pas 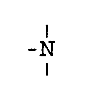 lorsque X est -S- et R est H;
n est 2, 3, 4 ou 5;
R est un atome d'hydrogène ou un groupe alkyle, alcoxy en C₁-C₆, hydroxy, carboxy, Cl, F, Br, I, un groupe amino, mono- ou dialkyl(C₁-C₆)amino, -NO₂, alkylthio inférieur, -OCF₃, cyano, acylamino, -CF₃, trifluoroacétyle, aminocarbonyle, 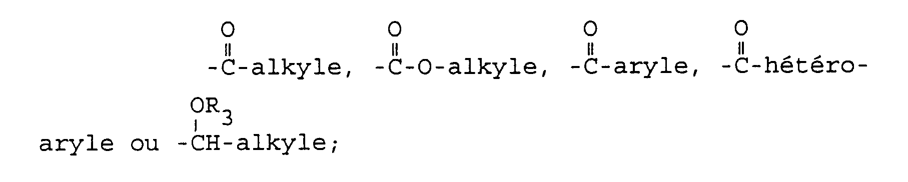
le groupe alkyle est un groupe alkyle inférieur;
le groupe aryle est le groupe phényle ou un groupe 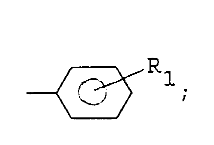 dans lequel R₁ est un atome d'hydrogène ou un groupe alkyle inférieur, alcoxy en C₁-C₆, hydroxy, Cl, F, Br, I, un groupe alkyl(C₁-C₆)amino, -NO₂, -CN, -CF₃, -OCF₃;
un groupe hétéroaryle est un groupe 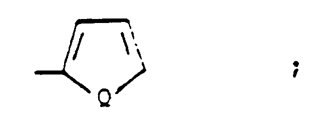
Q est -O-, -S-, 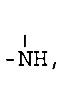 -CH=N-;
m est 1, 2 ou 3, étant entendu que lorsque m est 2 ou 3, les radicaux R peuvent être identiques ou différents;
R₃ est un atome d'hydrogène ou un groupe alkyle inférieur ou acyle;
ou d'un sel d'addition avec un acide pharmaceutiquement acceptable de celui-ci,
comprenant
a) la mise en réaction d'un composé de formule II 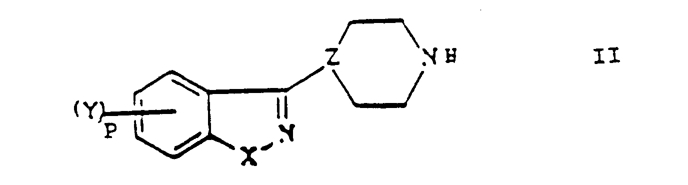 dans laquelle X, Y, Z et p sont tels que définis, dans des conditions d'alkylation, avec un composé de formule III 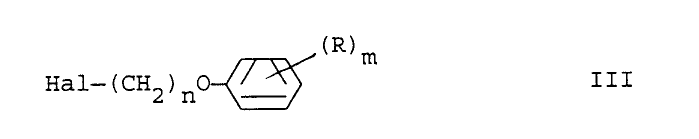 dans laquelle R, m et n sont tels que définis, et Hal est un atome de chlore, de brome ou d'iode,
b) éventuellement la réduction d'un composé de formule I dans lequel R est un groupe 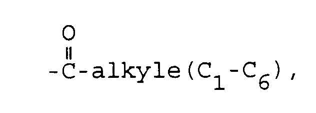 pour l'obtention d'un composé de formule I dans lequel R est un groupe CH₂(OH)-alkyle(C₁-C₆),
c) éventuellement la mise en réaction d'un composé de formule I dans lequel R est un groupe -CH(OR₃)-alkyle(C₁-C₆), dans lequel R₃ est un atome d'hydrogène, avec un anhydride d'acide alcanoïque, pour l'obtention d'un composé de formule I dans lequel R est un groupe alcanoyle en C₁-C₆,
d) éventuellement la mise en réaction d'un composé de formule I dans lequel R est le groupe hydroxy, d'abord avec une base forte et ensuite avec un composé de formule Hal-alkyle-(C₁-C₆), dans lequel Hal est un atome de chlore, de brome ou d'iode, pour l'obtention d'un composé de formule I dans lequel R est un groupe alcoxy en C₁-C₆,
e) éventuellement la mise en réaction d'un composé de formule I dans lequel X est N-R₂, et R₂ est un atome d'hydrogène, avec un halogénure d'alcanoyle en C₁-C₆ ou d'aroyle, pour l'obtention d'un composé de formule I dans lequel X est NR₂, et R₂ est un groupe alcanoyle en C₁-C₆ ou aroyle.

2. Procédé selon la revendication 1, dans lequel la réaction de l'étape a) est effectuée dans un solvant inerte, à une température dans la plage allant de 50°C à la température de reflux du solvant, en présence d'un capteur d'acide.

3. Procédé selon la revendication 2, dans lequel on utilise comme capteur d'acide un carbonate de métal alcalin.

4. Procédé selon la revendication 1, dans lequel on effectue la réduction selon l'étape b) en utilisant du borohydrure de sodium.

5. Procédé selon la revendication 1, dans lequel la base forte utilisée dans l'étape d) est l'hydrure de sodium.

6. Procédé selon la revendication 1, dans lequel X est -O-, -S- ou -NH-.

7. Procédé selon la revendication 1, dans lequel n est 2, 3 ou 4, Y est un atome d'hydrogène, de chlore ou de fluor en position 6 et p est 1.

8. Procédé selon la revendication 1, dans lequel R est un atome d'hydrogène ou d'halogène ou un groupe alkyle en C₁-C₆, alcoxy en C₁-C₆, alkyl(C₁-C₆)-thio, alkyl(C₁-C₆)-amino, 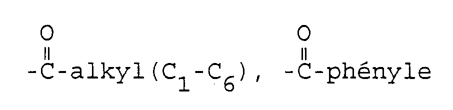 ou -CH(OR₃)-alkyle-(C₁-C₆), R₃ étant un atome d'hydrogène ou un groupe alkyle en C₁-C₆, et m est 2 ou 3.

9. Procédé selon la revendication 1, dans lequel n est 3 ou 4, m est 2 et les radicaux R sont -OCH₃ et

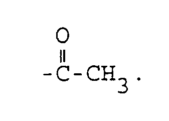

10. Procédé selon la revendication 1, dans lequel on prépare la 1-[4-[3-[4-(1H-indazol-3-yl)-1-pipérazinyl]propoxy]-3-méthoxyphényl]éthanone ou un sel d'addition avec un acide pharmaceutiquement acceptable de celle-ci.

11. Procédé selon la revendication 1, dans lequel on prépare la 1-[4-[3-[4-(1,2-benzisoxazol-3-yl)-1-pipéridinyl]-propoxy]-3-méthoxyphényl]éthanone ou un sel d'addition avec un acide pharmaceutiquement acceptable de celle-ci.

12. Composé selon la revendication 1, dans lequel on prépare la 1-[4-[3-[4-(6-fluoro-1,2-benzisoxazol-3-yl)-1-pipéridinyl]propoxy]-3-méthoxyphényl]éthanone ou un sel d'addition avec un acide pharmaceutiquement acceptable de celle-ci.

13. Composé selon la revendication 1, dans lequel on prépare la 1-[4-[4-[4-(1,2-benzisoxazol-3-yl)-1-pipéridinyl]butoxy]-3-méthoxyphényl]éthanone ou un sel d'addition avec un acide pharmaceutiquement acceptable de celle-ci.

14. Composé selon la revendication 1, dans lequel on prépare la 1-[4-[4-[4-(6-fluoro-1,2-benzisoxazol-3-yl)-1-pipéridinyl]butoxy]-3-méthoxyphényl]éthanone ou un sel d'addition avec un acide pharmaceutiquement acceptable de celle-ci.

15. Utilisation d'un composé de formule I tel que défini dans la revendication 1, pour la fabrication d'un médicament ayant une activité soit antipsychotique, soit antipsychotique et analgésique.
